# EUROPEAN PATENT SPECIFICATION

(11) **EP 4 582 018 B1**
(45) Date of publication and mention of the grant of the patent: **02.12.2026**
(21) Application number: 23894848.3
(22) Date of filing: 30.10.2023
(51) Int. Cl.: A61B 5/024, A61B 5/00

(54) **ELECTRONIC DEVICE INCLUDING SENSOR MODULE**
ELEKTRONISCHE VORRICHTUNG MIT SENSORMODUL
DISPOSITIF ÉLECTRONIQUE COMPRENANT UN MODULE DE CAPTEUR

(30) Priority: 23.11.2022 KR 20220158480; 20.12.2022 KR 20220179233
(43) Date of publication of application: 09.07.2025
(73) Proprietor: Samsung Electronics Co., Ltd., Suwon-si, Gyeonggi-do 16677 (KR)
(72) Inventor: YUN, Inho, Suwon-si Gyeonggi-do 16677 (KR); MIN, Eungi, Suwon-si Gyeonggi-do 16677 (KR); PARK, Minho, Suwon-si Gyeonggi-do 16677 (KR); AHN, Joongwoo, Suwon-si Gyeonggi-do 16677 (KR); LEE, Jeehoon, Suwon-si Gyeonggi-do 16677 (KR)
(74) Representative: Gulde & Partner
(86) International application number: PCT/KR2023/017025
(87) International publication number: WO 2024/111919

(56) References cited:
- WO-A1-2022/149850
- WO-A1-2022/215885
- KR-A- 20150 024 530
- KR-A- 20220 099 404
- KR-A- 20220 138 631
- US-A1- 2011 004 106
- US-A1- 2015 018 647
- US-A1- 2019 069 781
- US-A1- 2020 000 345
- US-A1- 2020 000 345
- US-A1- 2020 260 972
- US-A1- 2022 117 557
- US-A1- 2022 167 864

## Description

### BACKGROUND

### 1. Technical Field

Embodiments of the disclosure described herein relate to an electronic device including a sensor module.

### 2. Description of Related Art

Portable electronic devices can include smart phones, tablet PCs, wearable electronic devices, and the like. Moreover, it has become desirable to be able to obtain a user's biometric information. For example, the portable electronic device may provide various functions for health care based on the obtained biometric information.

The above-described information may be provided as related art for a better understanding of the disclosure. No claim or determination is made as to whether any of the above-described contents is applicable as prior art related to the disclosure.
US2022167864A1, US2020260972A1, WO2022149850A1, US2011004106A1, US2015018647A1, and WO2022215885A1 relates to wearable electronic devices with conventional optical sensor modules.

### SUMMARY

The present invention is defined by the appended set of claims. According to an embodiment of the disclosure, an electronic device comprises: a plurality of light emitting units spaced apart from each other to form a ring shape on a printed circuit board; at least one light receiving unit disposed between the plurality of light emitting units; and a processor configured to analyze light generated from the light emitting units and incident to the light receiving unit, wherein a separation distance between each of the plurality of light emitting units and the at least one light receiving unit is such that the at least one light receiving unit is configured to receive reflected light from light emitted at a beam angle from each of the plurality of light emitting units.

According to an embodiment of the disclosure, an electronic device comprises: a plurality of light emitting units disposed on a printed circuit board and spaced apart from each other to form a ring shape; at least one light receiving unit disposed between the plurality of light emitting units; and a processor configured to analyze light generated from the light emitting units and incident to the light receiving unit, wherein each of a first one or more light emitting units and a second one or more light emitting units included in the plurality of light emitting units includes at least one first light body configured to emit light in a specified wavelength band, wherein the first light body of the first one or more light and the first light body of the second one or more light emitting units have different beam angles, and wherein a separation distance between the first light body of the first one or more light emitting units and the light receiving unit is different from a separation distance between the first light body of the second one or more light emitting units and the light receiving unit.

According to an embodiment of the disclosure, a wearable electronic device includes a plurality of light emitting units spaced apart from each other to form a ring shape, at least one light receiving unit disposed between the plurality of light emitting units, and a processor that analyzes light generated from the light emitting units and incident to the light receiving unit, and a separation distance between each of the plurality of light emitting units and the light receiving unit varies depending on a beam angle of the light.

According to an embodiment of the disclosure, a method for operating an electronic device includes identifying a current state of an object to be measured on which the electronic device is worn, controlling driving of at least one of a plurality of light emitting units depending on the current state of the object to be measured, the plurality of light emitting units being spaced apart from each other to form a ring shape, and obtaining a light signal through a plurality of light receiving units disposed between the plurality of light emitting units. A separation distance between each of the plurality of light emitting units and the light receiving unit varies depending on a beam angle of the light.

According to an embodiment of the disclosure, a method for operating an electronic device includes obtaining light signals emitted from a plurality of light emitting units through a plurality of light receiving units when an object to be measured on which the electronic device is worn makes a big movement and obtaining a light signal emitted from a light having the smallest beam angle among the plurality of light emitting units through at least one light receiving unit disposed around the light having the smallest beam angle when the object to be measured makes a little movement.

### BRIEF DESCRIPTION OF THE DRAWINGS

FIG. 1 is a block diagram illustrating an electronic device in a network environment, according to an embodiment;
FIG. 2A is a front perspective view of an electronic device according to an embodiment, and FIG. 2B is a rear perspective view of the electronic device of FIG. 2A.
FIG. 3 is an exploded perspective view illustrating an electronic device according to an embodiment.
FIG. 4 is a schematic block diagram illustrating a biometric signal processing device according to an embodiment.
FIG. 5 is a view illustrating an arrangement of a sensor module of an electronic device according to an embodiment.
FIG. 6 is a view illustrating an arrangement of a sensor module of an electronic device according to an embodiment.
FIGS. 7A and 7B are views illustrating an arrangement of a sensor module of an electronic device according to an embodiment.
FIG. 8 is a view illustrating an arrangement of a sensor module of an electronic device according to an embodiment.
FIGS. 9A and 9B are views illustrating arrangements of sensor modules of electronic devices according to embodiments, and FIGS. 9C and 9D are views for explaining beam angles of light emitting units illustrated in FIGS. 9A and 9B.
FIGS. 10A, 10B, and 10C are views illustrating various examples of a light according to an embodiment.
FIGS. 11A to 11C are views illustrating examples of measuring biometric signals using an electronic device including a sensor module according to an embodiment.
FIG. 12 is a flowchart for explaining a method of identifying signal characteristics of a sensor module of an electronic device according to an embodiment.
FIG. 13 is a flowchart for explaining a method of identifying signal characteristics of a sensor module of an electronic device according to an embodiment.
FIG. 14 illustrates biometric signals obtained through an electronic device according to an embodiment.

With regard to description of the drawings, identical or similar reference numerals may be used to refer to identical or similar components.

### DETAILED DESCRIPTON

Hereinafter, certain embodiments of the disclosure may be described with reference to accompanying drawings. Accordingly, those of ordinary skill in the art will recognize that modification, equivalent, and/or alternative on the certain embodiments described herein can be variously made without departing from the scope of the disclosure.

Hereinafter, with reference to the drawings, embodiments of the present disclosure will be described in detail so that those skilled in the art can easily carry out the present disclosure. However, the present disclosure may be embodied in many different forms and is not limited to the embodiments described herein. In connection with the description of the drawings, the same or similar reference numerals may be used for the same or similar elements. Also, in the drawings and related descriptions, descriptions of well-known functions and constructions may be omitted for clarity and conciseness.

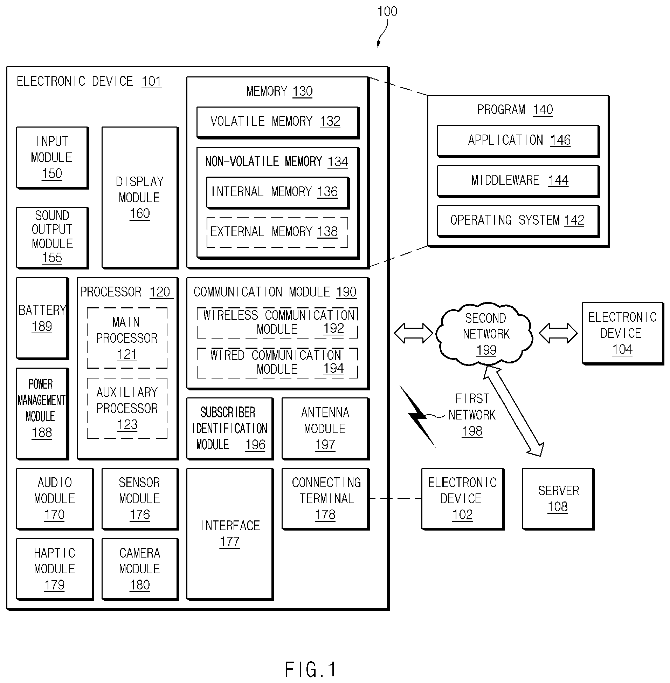

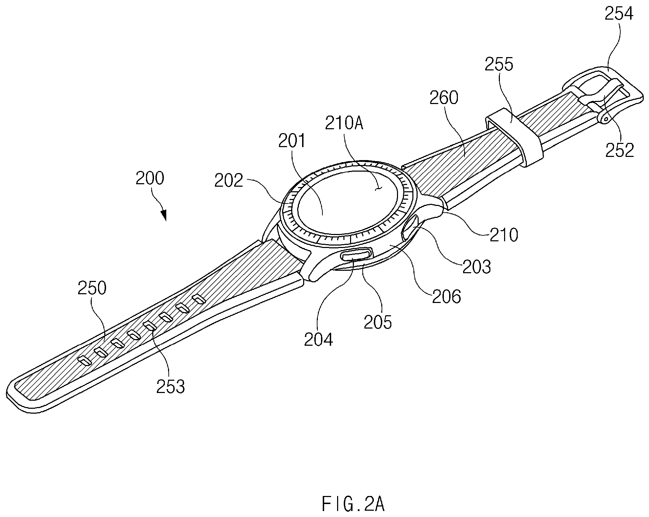

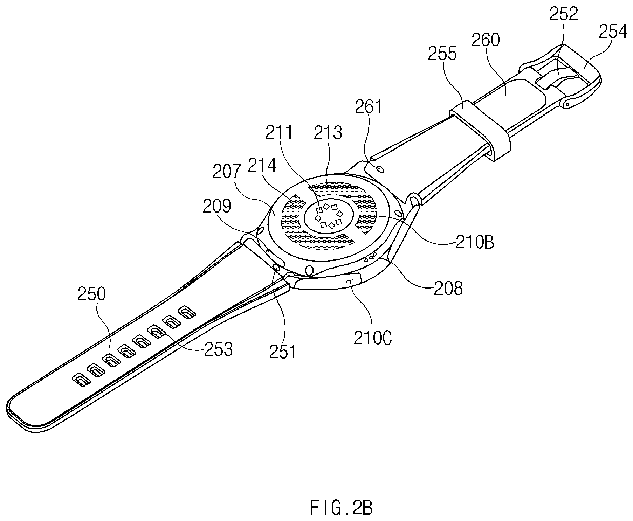

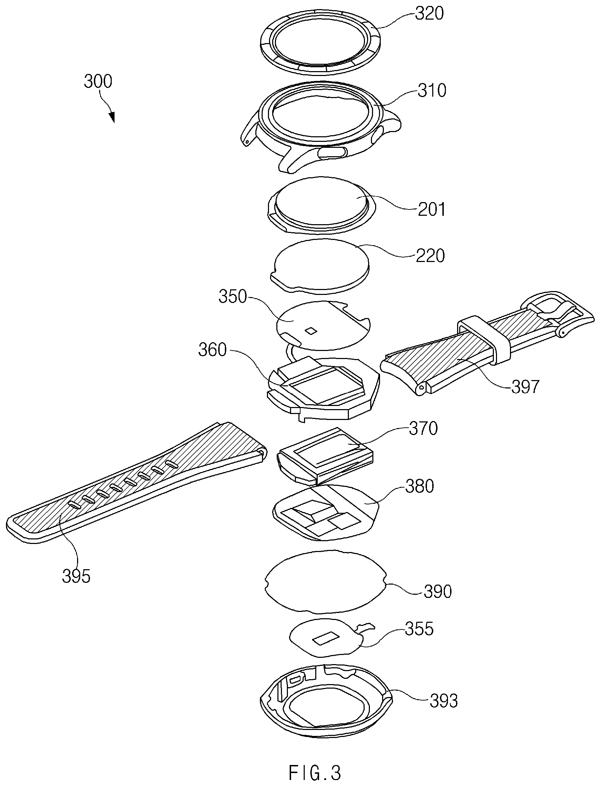

FIG. 1 describes an electronic device according to certain embodiments. FIGS. 2A, 2B, and 3 describe a housing of an electronic device, such as a smartwatch, according to certain embodiments.

### ELECTRONIC DEVICE

FIG. 1 is a block diagram illustrating an electronic device 101 in a network environment 100 according to certain embodiments.

Referring to FIG. 1, the electronic device 101 in the network environment 100 may communicate with an electronic device 102 via a first network 198 (e.g., a short-range wireless communication network), or an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, a memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one (e.g., the connecting terminal 178) of the components may be omitted from the electronic device 101, or one or more other components may be added in the electronic device 101. In some embodiments, some of the components may be implemented as single integrated circuitry. For example, some (e.g., the sensor module 176, the camera module 180, or the antenna module 197) of the components may be implemented as embedded in the display module 160 (e.g., a display).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may load a command or data received from another component (e.g., the sensor module 176 or the communication module 190) in a volatile memory 132, process the command or the data stored in the volatile memory 132, and store resulting data in a non-volatile memory 134. According to an embodiment, the processor 120 may include a main processor 121 (e.g., a central processing unit (CPU) or an application processor (AP)), and an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. When the electronic device 101 includes the main processor 121 and the auxiliary processor, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., a neural network processing device) may include a hardware structure specified for processing an artificial intelligence (AI) model. The AI model may be generated through machine learning. The learning may be performed by the electronic device 101 performing the AI, and may be performed through an additional server (e.g., the server 108). A learning algorithm may include, for example, a supervised learning algorithm, an unsupervised learning algorithm, a semi-supervised learning algorithm, or a reinforcement learning algorithm, but the disclosure is not limited thereto. The AI model may include a plurality of artificial neural network (ANN) layers. The ANN may include a deep neural network (DNN), a convolutional neural network (CNN), a recurrent neural network (RNN), a restricted boltzman machine (RBM), a deep belief network (DBN), a bidirectional recurrent deep neural network (BRDNN), a deep Q-networks or the combination of the above networks, but the disclosure is not limited thereto. The AI model may additionally or alternatively include a software structure, in addition to a hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by other component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record, and the receiver may be used for an incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or an external electronic device (e.g., the electronic device 102) (e.g., speaker of headphone) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). The corresponding communication module from among the communication modules may communicate with the external electronic device via the first network 198 (e.g., a short-range communication network, such as Bluetooth^{™}, wireless-fidelity (WiFi) direct, or infrared data association (IrDA)) or the second network 199 (e.g., a long-range communication network, such as a legacy cellular network, 5G network, next generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify or authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network and a next-generation communication technology, for example, a new radio (NR) access technology after a 4G network. The NR access technology may support high-speed transmission for high capacity data (enhanced mobile broadband; eMBB), terminal power minimizing and multiple terminal access (massive machine type communication; mMTC), or ultra-reliable and low-latency communications (URLLC). The wireless communication module 192 may support a high-frequency band (e.g., mmWave band) to achieve, for example, a higher data rate. The wireless communication module 192 may support various technologies, for example, beamforming, massive multiple-input and multiple-output (MIMO), Full-dimensional MIMO, an array antenna, analog beam-forming, or a large-scale antenna, to secure performance in high frequency bands. The wireless communication module 192 may support various requirements defined in the electronic device 101, the external electronic device (e.g., the electronic device 104) or the network system (e.g., the second network 199). According to one embodiment, the wireless communication module 192 may support a peak data rate (e.g., 20Gbps or more) for eMBB realization, loss coverage (e.g., 164 dB or less) for mMTC realization, or U-plane latency (e.g., 0.5 ms or less, or the round trip of 1 ms or less in each of a downlink (DL) and an uplink (UL)) for URLCC realization.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., PCB). According to an embodiment, the antenna module 197 may include a plurality of antennas (e.g., an array antenna). In such a case, at least one antenna appropriate for a communication scheme used in the communication network, such as the first network 198 or the second network 199, may be selected, for example, by the communication module 190 from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to certain embodiments, the antenna module 197 may form an mmWave antenna module. According to an embodiment, the mmWave antenna module may include a printed circuit board, an RFIC disposed on a first surface (e.g., a bottom surface) of the printed circuit board, or disposed adjacent to the first surface to support the specific high frequency band (e.g., mmWave band), and a plurality of antennas (e.g., an array antenna) disposed on a second surface (e.g., a top surface or a side surface) of the printed circuit board or disposed adjacent to the second surface to transmit or receive a signal having the specified high frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the external electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, when the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide an ultra-latency service by using, for example, distributed computing or mobile edge computing. According to certain embodiments, the external electronic device 104 may include the Internet of things (IoT). The server 108 may be an artificial server using machine learning and/or a neural network. According to an embodiment, the external electronic device 104 or the server 108 may be included in the second network 199. The electronic device 101 may be applied to an artificial intelligence service (e.g., a smart home, a smart city, a smart car, or healthcare service) based on the 5G communication technology and the IoT-related technology.

In certain embodiments, the sensor module 176 can be configured to take biometric measurements. The biometric measurements can be used for healthcare application. Moreover, the electronic device 100 can comprise a wearable electronic device such as a smartwatch. The smartwatch can be worn on the wrist, and the sensor module 176 may be proximate or make contact with the wrist.

### HOUSING

FIG. 2A is a front perspective view of an electronic device according to an embodiment, and FIG. 2B is a rear perspective view of the electronic device of FIG. 2A.

Referring to FIGS. 2A and 2B, the electronic device 200 according to an embodiment (e.g., the electronic device 101 of FIG. 1) may include a housing 210 and fastening members 250 and 260. The housing 210 may include a first surface (or, a front surface) 210A, a second surface (or, a rear surface) 210B, and a side surface 210C surrounding a space between the first surface 210A and the second surface 210B. The fastening members 250 and 260 may be connected to at least portions of the housing 210 and may detachably fasten the electronic device 200 around a part (e.g., a wrist, an ankle, or the like) of a user's body. In another embodiment (not illustrated), the housing may also refer to a structure that forms a part of the first surface 210A, the second surface 210B, and the side surface 210C of FIG. 2A. According to an embodiment, the first surface 210A may be formed by a front plate 201, at least a portion of which is substantially transparent (e.g., a glass plate including various coating layers, or a polymer plate). The second surface 210B may be formed by a back plate 207 that is substantially opaque. The back plate 207 may be formed of, for example, coated or colored glass, ceramic, a polymer, metal (e.g., aluminum, stainless steel (STS), or magnesium), or a combination of at least two of the aforementioned materials. The side surface 210C may be formed by a side bezel structure (or, a "side member") 206 that is coupled with the front plate 201 and the back plate 207 and that includes metal and/or a polymer. In some embodiments, the back plate 207 and the side bezel structure 206 may be integrally formed with each other and may include the same material (e.g., a metallic material such as aluminum). The fastening members 250 and 260 may be formed of various materials and may have various forms. The fastening members 250 and 260 may be formed of woven fabric, leather, rubber, urethane, metal, ceramic, or a combination of at least two of the aforementioned materials. The fastening members 250 and 260 may be implemented in an integrated form or with a plurality of unit links that are movable relative to each other.

According to an embodiment, the electronic device 200 may include at least one of a display 220 (refer to FIG. 3), audio modules 205 and 208, a sensor module 211, key input devices 202, 203, and 204, or a connector hole 209. In some embodiments, the electronic device 200 may not include at least one component (e.g., the key input devices 202, 203, and 204, the connector hole 209, or the sensor module 211) among the aforementioned components, or may additionally include other component(s).

The display 220, for example, may be exposed through most of the front plate 201. The display 220 may have a shape corresponding to the shape of the front plate 201. The display 220 may have various shapes such as a circular shape, an oval shape, a polygonal shape, or the like. The display 220 may be combined with, or disposed adjacent to, touch detection circuitry, a pressure sensor capable of measuring the intensity (pressure) of a touch, and/or a fingerprint sensor.

The audio modules 205 and 208 may include the microphone hole 205 and the speaker hole 208. A microphone for obtaining external sound may be disposed in the microphone hole 205, and in some embodiments, a plurality of microphones may be disposed in the microphone hole 205 to sense the direction of sound. The speaker hole 208 may be used for an external speaker and a receiver for telephone call. In some embodiments, the speaker hole 208 and the microphone hole 205 may be implemented as a single hole, or a speaker (e.g., a piezoelectric speaker) may be included without the speaker hole 208.

The sensor module 211 may generate an electrical signal or a data value that corresponds to an operational state inside the electronic device 200 or an environmental state external to the electronic device 200. The sensor module 211 may include, for example, a biosensor module 211 (e.g., a heart rate monitor (HRM) sensor) disposed on the second surface 210B of the housing 210. The electronic device 200 may further include a non-illustrated sensor module, which may be, for example, at least one of a gesture sensor, a gyro sensor, an atmospheric pressure sensor, a magnetic sensor, an acceleration sensor, a grip sensor, a color sensor, an infrared (IR) sensor, a biosensor, a temperature sensor, a humidity sensor, or an illuminance sensor.

The sensor module 211 may include electrode regions 213 and 214 that form portions of a surface of the electronic device 200 and biometric signal detection circuitry (not illustrated) that is electrically connected with the electrode regions 213 and 214. For example, the electrode regions 213 and 214 may include the first electrode region 213 and the second electrode region 214 that are disposed on the second surface 210B of the housing 210. The sensor module 211 may be configured such that the electrode regions 213 and 214 obtain electrical signals from a part of the user's body and the biometric signal detection circuitry detects biometric information of the user based on the electrical signals.

The key input devices 202, 203, and 204 may include the wheel key 202 disposed on the first surface 210A of the housing 210 so as to be rotatable in at least one direction and/or the side key buttons 203 and 204 disposed on the side surface 210C of the housing 210. The wheel key may have a shape corresponding to the shape of the front plate 201. In another embodiment, the electronic device 200 may not include all or some of the aforementioned key input devices 202, 203, and 204, and the key input devices 202, 203, and 204 not included may be implemented in a different form, such as a soft key, on the display 220. The connector hole 209 may include another connector hole (not illustrated) that is capable of accommodating a connector (e.g., a USB connector) for transmitting and receiving electric power and/or data with an external electronic device and is capable of accommodating a connector for transmitting and receiving audio signals with an external electronic device. The electronic device 200 may further include, for example, a connector cover (not illustrated) that covers at least a portion of the connector hole 209 and blocks infiltration of external foreign matter into the connector hole. In another embodiment, the electronic device 200 may not include all or a part of the connector hole 209 and the connector cover.

The fastening members 250 and 260 may be detachably fastened to at least partial regions of the housing 210 by using locking members 251 and 261. The fastening members 250 and 260 may include at least one of a fixing member 252, fixing member fastening holes 253, a band guide member 254, or a band fixing ring 255.

The fixing member 252 may be configured to fix the housing 210 and the fastening members 250 and 260 to a part (e.g., a wrist, an ankle, or the like) of the user's body. The fixing member fastening holes 253 may fix the housing 210 and the fastening members 250 and 260 to the part of the user's body to correspond to the fixing member 252. The band guide member 254 may be configured to restrict a movement range of the fixing member 252 when the fixing member 252 is fastened to one of the fixing member fastening holes 253. Accordingly, the fastening members 250 and 260 may be fastened around the part of the user's body in a state of being brought into close contact with the part of the user's body. The band fixing ring 255 may limit a movement range of the fastening members 250 and 260 in the state in which the fixing member 252 is fastened to one of the fixing member fastening holes 253. In another embodiment, the fastening members 250 and 260 may not include at least one of the fixing member 252, the fixing member fastening holes 253, the band guide member 254, or the band fixing ring 255. For example, the fastening members 250 and 260 may be coupled with each other and formed in a ring shape. Accordingly, the fastening members 250 and 260 may not include the fixing member 252, the fixing member fastening holes 253, the band guide member 254, and the band fixing ring 255.

FIG. 3 is an exploded perspective view illustrating an electronic device according to an embodiment.

Referring to FIG. 3, the electronic device 300 (e.g., the electronic device 101 of FIG. 1 or the electronic device 200 of FIG. 2) may include a side bezel structure 310 (e.g., the side bezel structure 206 of FIG. 2A), a wheel key 320 (e.g., the wheel key 202 of FIG. 2A), the front plate 201, the display 220, a first antenna 350, a second antenna 355, a support member 360 (e.g., a bracket), a battery 370, a printed circuit board 380, a sealing member 390, a back plate 393 (e.g., the back plate 207 of FIG. 2B), and fastening members 395 and 397 (e.g., the fastening members 250 and 260 of FIGS. 2A and 2B). At least one of the components of the electronic device 300 may be identical or similar to at least one of the components of the electronic device 200 of FIGS. 2A and 2B, and repetitive descriptions will hereinafter be omitted. The support member 360 may be disposed inside the electronic device 300 and may be connected with the side bezel structure 310, or may be integrally formed with the side bezel structure 310. The support member 360 may be formed of, for example, a metallic material and/or a nonmetallic (e.g., polymer) material. The display 220 may be coupled to one surface of the support member 360, and the printed circuit board 380 may be coupled to an opposite surface of the support member 360. The printed circuit board 380 may have a processor, a memory, and/or an interface mounted thereon. The processor may include, for example, one or more of a central processing unit, an application processor, a graphic processing unit (GPU), a sensor processor, or a communication processor.

The memory may include, for example, a volatile memory or a nonvolatile memory. The interface may include, for example, a high definition multimedia interface (HDMI), a universal serial bus (USB) interface, an SD card interface, and/or an audio interface. For example, the interface may electrically or physically connect the electronic device 300 with an external electronic device and may include a USB connector, an SD card/MMC connector, or an audio connector.

The battery 370 may be a device for supplying electric power to at least one component of the electronic device 300. The battery 370 may include, for example, a primary cell that is not rechargeable, a secondary cell that is rechargeable, or a fuel cell. At least a portion of the battery 370, for example, may be disposed on substantially the same plane as the printed circuit board 380. The battery 370 may be integrally disposed inside the electronic device 300, or may be disposed so as to be detachable from the electronic device 300.

The first antenna 350 may be disposed between the display 220 and the support member 360. The first antenna 350 may include, for example, a near field communication (NFC) antenna, a wireless charging antenna, and/or a magnetic secure transmission (MST) antenna. The first antenna 350, for example, may perform short-range communication with an external device, or may wirelessly transmit and receive electric power required for charging, and may transmit a magnetism-based signal including a short-range communication signal or payment data. In another embodiment, an antenna structure may be formed by a portion of the side bezel structure 310 and/or a portion of the support member 360, or a combination thereof.

The second antenna 355 may be disposed between the printed circuit board 380 and the back plate 393. The second antenna 355 may include, for example, a near field communication (NFC) antenna, a wireless charging antenna, and/or a magnetic secure transmission (MST) antenna. The second antenna 355, for example, may perform short-range communication with an external device, or may wirelessly transmit and receive electric power required for charging, and may transmit a magnetism-based signal including a short-range communication signal or payment data. In another embodiment, an antenna structure may be formed by a portion of the side bezel structure 310 and/or a portion of the back plate 393, or a combination thereof.

The sealing member 390 may be located between the side bezel structure 310 and the back plate 393. The sealing member 390 may be configured to block moisture and foreign matter introduced from the outside into a space surrounded by the side bezel structure 310 and the back plate 393.

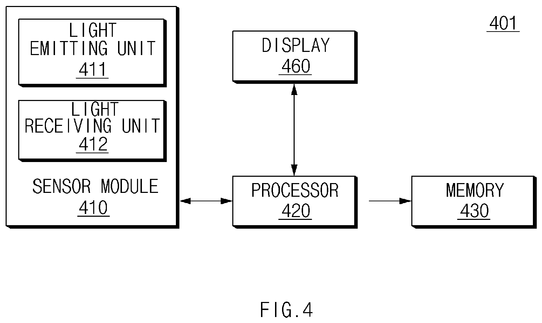

FIG. 4 is a schematic block diagram illustrating a biometric signal processing device according to an embodiment.

Referring to FIG. 4, the biometric signal processing device 401 may be a wearable electronic device (e.g., the electronic device of FIG. 1, the electronic device 200 of FIGS. 2A and 2B, or the electronic device 300 of FIG. 3). Without being limited thereto, the biometric signal processing device 401 may be a portable terminal such as a smart phone or a tablet computer, a patch or sticker type device, or an implantable device.

For example, the biometric signal processing device 401 may be equipped in a wearable electronic device in the form of a hardware or software module. In another example, the biometric signal processing device 401 may be implemented as an independent hardware device. The biometric signal processing device 401 may be used to obtain and analyze various types of biometric signals. However, without being limited thereto, various changes and modifications may be made depending on the purpose of use of the technology described in the disclosure.

The biometric signal processing device 401 may include a sensor module 410 (e.g., the sensor module 176 of FIG. 1 or/and the sensor module 211 of FIG. 2B), a processor 420 (e.g., the processor 120 of FIG. 1), a memory 430 (e.g., the memory 130 of FIG. 1), and a display 460 (e.g., the display module 160 of FIG. 1 or/and the display 220 of FIG. 3).

The sensor module 410 may include a plurality of sensors and may receive, through the plurality of sensors, signals for obtaining biometric signals. For example, the plurality of sensors may optionally include another sensor (e.g., an acceleration sensor) for measuring a necessary biometric signal as needed as well as a biosensor such as an electrocardiogram (ECG) sensor, a photoplethysmograph (PPG) sensor, a heart rate monitor sensor, or a body temperature sensor. For example, among the plurality of sensors, the PPG sensor is a sensor that applies light to a user's body (or, the user's skin) and estimates various biometric states based on characteristics of the body or the blood flow inside the body by using the property that the light is absorbed, scattered, or reflected in the skin tissue of the user's body.

According to an embodiment, at least one PPG sensor may include at least two light emitting units 411 (or, light sources) and at least one light receiving unit (or, light detector, or photosensor) 412. Light in a specific wavelength band that is output from the light emitting units 411 may be applied to the user's body. The light emitting units 411 may apply light having constant intensity to the user's body, and the wavelength of the applied light may vary depending on the purpose of measurement or the type of target component to be analyzed. For example, the light emitting units 411 may include at least one light including a light emitting diode (LED) or a laser diode (LD). The light emitting units 411 may use light in various wavelength bands such as green light, red light, blue light, or infrared light for a reduction of an influence of motion artifacts. The light emitting units 411 may emit light by sequentially or simultaneously turning on or crossing light in several wavelength bands.

The sensor module 410 may detect, through the light receiving unit 412, light diffusely reflected from the user's body to correspond to the applied light. The sensor module 410 may output a biometric signal corresponding to the reflected light returning from the user's body through the light receiving unit 412.

The light receiving unit 412 of the sensor module 410 may receive at least a portion of the returning reflected light and may generate at least one piece of biometric information using an electrical signal obtained by converting the light. For example, the light receiving unit 412 may include a photodiode (PD), a photo transistor, or a charge-coupled device (CCD). However, the light receiving unit 412 is not limited to the foregoing, and can comprise an element capable of converting a light signal into an electrical signal. The light receiving unit 412 may have a reflective or transmissive structure.

The sensor module 410 may receive electric current corresponding to a light signal received through the light receiving unit 412, may convert the received current signal into a digital signal, and may transfer the digital signal to the processor 420. The sensor module 410 may perform current-voltage conversion for processing the light signal received through the light receiving unit 412, may digitize an output analog voltage signal before transferring the output analog voltage signal to the processor 420, and may transfer the digitized signal to the processor 420. Based on a biometric signal from the sensor module 410, the processor 420 may analyze the biometric signal.

The sensor module 410 may further include a motion sensor (not illustrated). The motion sensor may include various types of sensors capable of sensing movement of the biometric signal processing device 401. For example, the motion sensor may include a gyro sensor, an acceleration sensor, or a terrestrial magnetism sensor. The motion sensor may obtain at least one sensing signal that varies depending on movement of the user having the biometric signal processing device 401 on. Based on the sensing signal of the motion sensor, the biometric signal processing device 401 may determine the degree of movement of the biometric signal processing device 401 and the user having the biometric signal processing device 401 on.

The processor 420 may be the processor 120 of FIG. 1. Alternatively, the processor 420 may be a sensor processor implemented separately from the processor 120 of FIG. 1. For example, the processor 420 may measure a blood flow rate in a blood vessel, which is increased or decreased by heart beat, based on a biometric signal collected through the light receiving unit 412 and may measure pulse waves of the user based on the measured blood flow rate. For example, the processor 420 may obtain information about a heart rate of the user and may monitor a health condition based on the received information about the heart rate.

The processor 420 may detect, predict, or analyze a health condition of the user of the biometric signal processing device, based on a biometric signal from the sensor module 410. For example, the processor 420 may measure a heart rate using the PPG sensor of the sensor module 410, and the heart rate, which is the frequency of the heartbeat measured by the number of contractions of the heart per minute, may be used to determine a state of health at ordinary time or an exercise state. According to an embodiment, the processor 420 may determine stress or the level of tension using heart rate variability (HRV) based on heart rate interval information.

The processor 420 may analyze a biometric signal. The processor 420 may store the biometric signal in the memory 430 and may provide at least one piece of biometric information. Here, the at least one piece of biometric information may be information such as heart rate, blood pressure, or sleep apnea. The biometric information may be used as basic data for analyzing the user's physical strength or health condition.

The processor 420 may determine at least one of the intensity of light or an irradiation range, based on user-related biometric information stored in the memory 430 or information obtained through test driving of the light. For each user having the biometric signal processing device 401 on, skin tones and the structures and positions of capillaries may differ from one another depending on wearing positions. Accordingly, the processor 420 may select a light 411 to be turned on/off among a plurality of light emitting units 411 depending on the body structure of the user having the biometric signal processing device 401 on and may selectively determine the intensity of light of the turned-on light 411. The processor 420 may select a light to be turned on among the plurality of light emitting units depending on at least one of a required light emission range, light intensity, or position and may determine the intensity of light of the selected light.

The memory 430 may store data (e.g., biometric information) of the biometric signal processing device 401. For example, the memory 430 may store the structure of the user's capillary, the position of the user's capillary, and at least one of the skin tone, the blood pressure, the blood glucose level, the heart rate, the blood oxygen saturation, the stress index, or the body fat index of the user.

The display 460 may be implemented to be substantially the same as, or similar to, the display module 160 described with reference to FIG. 1. The display 460 may receive at least one piece of biometric information from the processor 420 and may visually display the biometric information. For example, the display 460 may display a user interface based on a biometric signal measured when an application for measurement of a biometric signal (e.g., an electrocardiogram applications or a health care application) is executed. In addition, under the control of the processor 420, the display 460 may output a guide screen or an abnormal state when a biometric signal is measured.

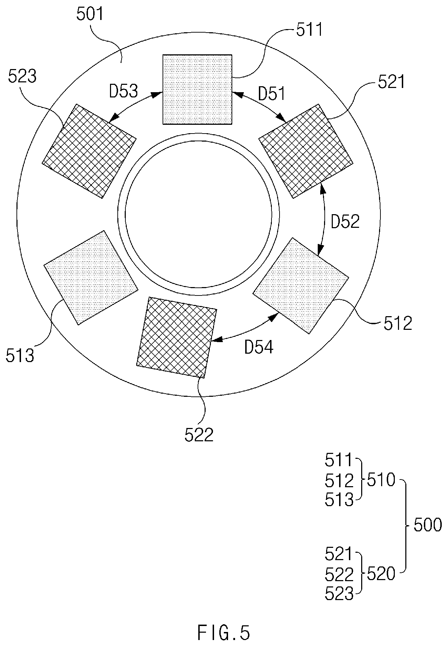

FIG. 5 is a view illustrating an arrangement of a sensor module of an electronic device according to an embodiment.

Referring to FIG. 5, the sensor module 500 may be mounted on a printed circuit board 501. The sensor module 500 may include a plurality of light emitting units 510 and a plurality of light receiving units 520. The number of light emitting units 510 may be equal to or different from the number of light receiving units 520. The light emitting units 510 and the light receiving units 520 included in the sensor module 500 may be arranged in a one-to-one or one-to-many correspondence. For example, the light emitting units 510 and the light receiving units 520 may be alternately arranged, physically spaced apart from each other. The light emitting units 510 and the light receiving units 520 may be substantially arranged in a ring shape including separation gaps.

Each one of the light emitting units 511, 512, and 513 can either sequentially or simultaneously emit light. The light from the light emitting units 511, 512, and 513 is transmitted to the user's body, and reflected. The two light receiving units that are adjacent to each light light emitting units are configured to detect the reflected light.

For example, the first light emitting unit 511, the second light emitting unit 512, and the third light emitting unit 513 may sequentially emit light in a specified order, or may simultaneously emit light.

At least one of the first light receiving unit 521 or the third light receiving unit 523 (disposed adjacent to the first light emitting unit 511) may form a first optical sensor together with the first light emitting unit 511. At least one of the first light receiving unit 521 or the third light receiving unit 523 may have a light receiving wavelength band that corresponds to the emission wavelength of the first light emitting unit 511. At least one of the first light receiving unit 521 or the third light receiving unit 523 may obtain at least a portion of light output through the first light emitting unit 511 and then reflected by a user's body.

At least one of the first light receiving unit 521 or the second light receiving unit 522 (disposed adjacent to the second light emitting unit 512) may form a second optical sensor together with the second light emitting unit 512. At least one of the first light receiving unit 521 or the second light receiving unit 522 may have a light receiving wavelength band that corresponds to the emission wavelength of the second light emitting unit 512. At least one of the first light receiving unit 521 or the second light receiving unit 522 may obtain at least a portion of light output through the second light emitting unit 512 and reflected by the user's body.

At least one of the second light receiving unit 522 or the third light receiving unit 523 disposed adjacent to the third light emitting unit 513 may form a third optical sensor together with the third light emitting unit 513. At least one of the second light receiving unit 522 or the third light receiving unit 523 may have a light receiving wavelength band that corresponds to the emission wavelength of the third light emitting unit513. At least one of the second light receiving unit 522 or the third light receiving unit 523 may obtain at least a portion of light output through the third light emitting unit513 and reflected by the user's body.

The sensor module 500 may be arranged in the order of the first light emitting unit 511, the first light receiving unit 521, the second light emitting unit 512, the second light receiving unit 522, the third light emitting unit 513, and the third light receiving unit 523. The first light emitting unit 511, the first light receiving unit 521, the second light emitting unit 512, the second light receiving unit 522, the third light emitting unit 513, and the third light receiving unit 523 may be spaced apart from each other in the clockwise or counterclockwise direction.

At least one of the separation distances between the plurality of light emitting units 510 and the plurality of light receiving units 520 may be different from the remaining separation distances. For example, the separation distances between the plurality of light emitting units 510 and the plurality of light receiving units 520 may be different from one another. In another example, some of the separation distances between the plurality of light emitting units 510 and the plurality of light receiving units 520 may be the same as one another, and the remaining separation distances may be different from one another.

For example, the first light receiving unit 521 may be spaced apart from the first light emitting unit 511 and the second light emitting unit 512 (disposed on the opposite side of the first light receiving unit 521) by different distances. The first light receiving unit 521 may be spaced apart from the first light emitting unit 511 by a first separation distance D51 and may be spaced apart from the second light emitting unit 512 by a second separation distance D52. The second separation distance D52 may different from the first separation distance D51.

The first light emitting unit 511 may be spaced apart from the first light receiving unit 521 and the third light receiving unit 523 disposed on opposite sides of the first light emitting unit 511 by specified distances. The first light emitting unit 511 may be spaced apart from the first light receiving unit 521 by the first separation distance D51 and may be spaced apart from the third light receiving unit 523 by a third separation distance D53. The third separation distance D53 may equal to, substantially equal to, or similar to, the first separation distance D51. At least one of the first separation distance D51 or the third separation distance D53 may be different from the second separation distance D52.

The second light emitting unit 512 may be spaced apart from the first light receiving unit 521 and the second light receiving unit 522 (disposed on the opposite side of the second light emitting unit 512) by specified distances. The second light emitting unit 512 may be spaced apart from the first light receiving unit 521 by the second separation distance D52 and may be spaced apart from the second light receiving unit 522 by a fourth separation distance D54. The fourth separation distance D54 may be equal to, substantially equal to, or similar to, the second separation distance D52. At least one of the second separation distance D52 or the fourth separation distance D54 may be different from the first separation distance D51.

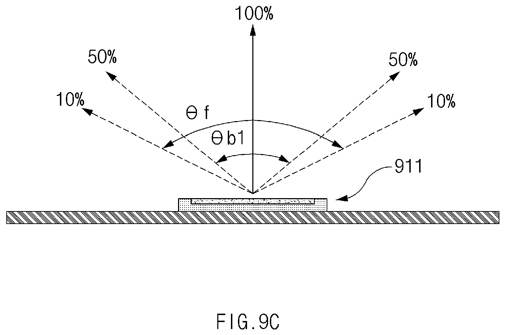

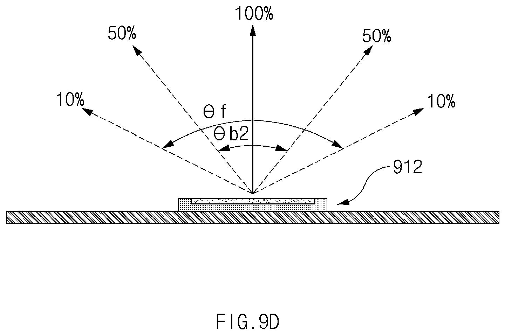

The separation distances between the plurality of light emitting units 510 and the plurality of light receiving units 520 may be set based on beam angles of the plurality of light emitting units 510 (e.g., beam angles θb1 and θb2 of FIGS. 9C and 9D). For example, the separation distances between the plurality of light emitting units 510 and the light receiving units 520 may be set to be proportional to the beam angles of the light sources 510. For example, as the beam angles of the light emitting units 510 are increased, the separation distances between the light emitting units 510 and the light receiving units 520 may be set to larger separation distances. As the beam angles of the light emitting units 510 are decreased, the separation distances between the light emitting units 510 and the light receiving units 520 may be set to smaller separation distances.

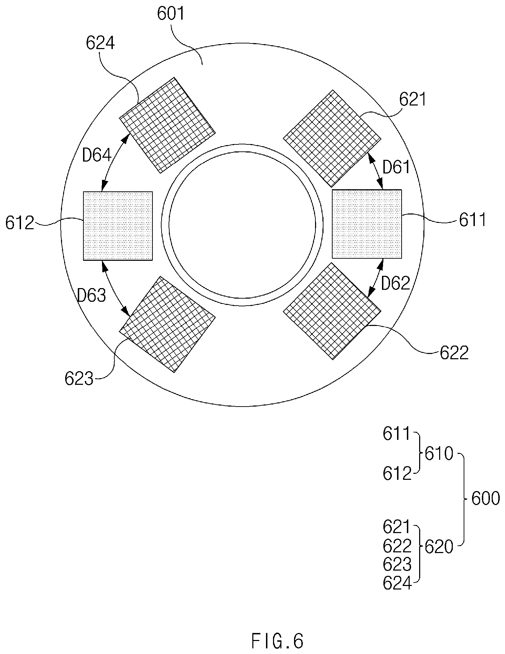

FIG. 6 is a view illustrating an arrangement of a sensor module of an electronic device according to an embodiment. In certain embodiments, there may be a different number of light receiving units 620 from light emitting units 610. For example, there may be four light receiving units 621, 622, 623, and 624, and two light emitting units 611, 612. The separation distances D63 and D64 between light emitting unit 612, and adjacent light receiving units 623 and 624 may be substantially the same. The separation distances D61 and D62 between light emitting unit 611 and adjacent light receiving units 621 and 622 may be substantially the same. The separation distances D61 and D62 may be substantially different from separation distances D63 and D64.

Referring to FIG. 6, the sensor module 600 mounted on a printed circuit board 601 may include a plurality of light emitting units 610 and a plurality of light receiving units 620. The number of light emitting units 610 may be different from the number of light receiving units. The number of light emitting units 610 may be greater than the number of light receiving units 620, or the number of light receiving units 620 may be greater than the number of light emitting units 610. The light emitting units 610 may be arranged to correspond to the light receiving units 620 in a one-to-many manner.

According to an embodiment, the light emitting units 610 may include a first light emitting unit 611 and a second light emitting unit 612. The first light emitting unit 611 and the second light emitting unit 612 may sequentially emit light in a specified order, or may simultaneously emit light. The light receiving units 620 may include a first light receiving unit 621, a second light receiving unit 622, a third light receiving unit 623, and a fourth light receiving unit 624. The first light receiving unit 621 and the second light receiving unit 622 may be disposed adjacent to the first light emitting unit 611 to form a first optical sensor. The first light receiving unit 621 and the second light receiving unit 622 may be located on opposite sides of the first light emitting unit 611 with the first light emitting unit 611 therebetween. The first light receiving unit 621 and the second light receiving unit 622 may have a light receiving wavelength band that corresponds to the emission wavelength of the first light emitting unit 611. The first light receiving unit 621 and the second light receiving unit 622 may have a light receiving wavelength band that corresponds to light in the same wavelength band or may have light receiving wavelength bands that correspond to light in different wavelength bands. For example, the first light receiving unit 621 may have a light receiving wavelength band that corresponds to the emission wavelength of one light included in the first light emitting unit 611. The second light receiving unit 622 may have a light receiving wavelength band that corresponds to the emission wavelength of another light included in the first light emitting unit 611. The first light receiving unit 621 and the second light receiving unit 622 may obtain at least a portion of light output through the first light emitting unit 611 and reflected by a user's body. The third light receiving unit 623 and the fourth light receiving unit 624 may be disposed adjacent to the second light emitting unit 612 to form a second optical sensor. The third light receiving unit 623 and the fourth light receiving unit 624 may be located on opposite sides of the second light emitting unit 612 with the second light emitting unit 612 therebetween. The third light receiving unit 623 and the fourth light receiving unit 624 may have a light receiving wavelength band that corresponds to the emission wavelength of the second light emitting unit 612. The third light receiving unit 623 and the fourth light receiving unit 624 may have a light receiving wavelength band that corresponds to light in the same wavelength band or may have light receiving wavelength bands that correspond to light in different wavelength bands. For example, the third light receiving unit 623 may have a light receiving wavelength band that corresponds to the emission wavelength of one light included in the second light emitting unit 612. The fourth light receiving unit 624 may have a light receiving wavelength band that corresponds to the emission wavelength of another light included in the second light emitting unit 612. The third light receiving unit 623 and the fourth light receiving unit 624 may obtain at least a portion of light output through the second light emitting unit 612 and reflected by the user's body.

The sensor module 600 may be arranged in the order of the first light receiving unit 621, the first light emitting unit 611, the second light receiving unit 622, the third light receiving unit 623, the second light emitting unit 612, and the fourth light receiving unit 624. The first light receiving unit 621, the first light emitting unit 611, the second light receiving unit 622, the third light receiving unit 623, the second light emitting unit 612, and the fourth light receiving unit 624 may be spaced apart from each other in the clockwise or counterclockwise direction.

According to an embodiment, at least one of the separation distances between the plurality of light emitting units 610 and the plurality of light receiving units 620 may be different from the remaining separation distances. For example, the separation distances between the plurality of light emitting units 610 and the plurality of light receiving units 620 may be different from one another. In another example, some of the separation distances between the plurality of light emitting units 610 and the plurality of light receiving units 620 may be the same as one another, and the remaining separation distances may be different from one another.

For example, the first light emitting unit 611 may be spaced apart from the first light receiving unit 621 by a first separation distance D61 and may be spaced apart from the second light receiving unit 622 by a second separation distance D62 that is substantially the same as the first separation distance D61.

The second light emitting unit 612 may be spaced apart from the third light receiving unit 623 by a third separation distance D63 and may be spaced apart from the fourth light receiving unit 624 by a fourth separation distance D64 that is substantially the same as the third separation distance D63. Each of the third separation distance D63 and the fourth separation distance D64 may be different from one of the first separation distance D61 and the second separation distance D62. According to an embodiment, the separation distances between the plurality of light emitting units 610 and the plurality of light receiving units 620 may be set based on beam angles of the plurality of light emitting units 610 (e.g., the beam angles θb1 and θb2 of FIGS. 9C and 9D). For example, the separation distances between the light emitting units 610 and the light receiving units 620 may be set to be proportional to the beam angles of the light emitting units 610.

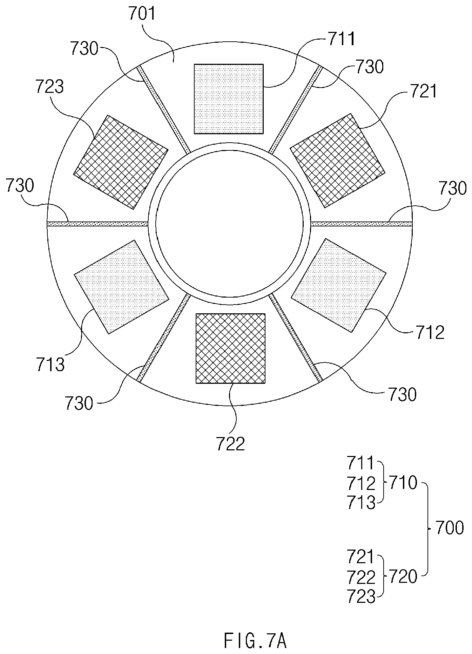

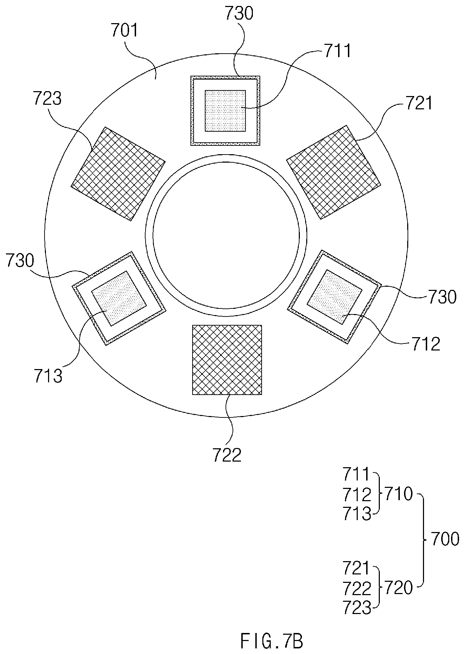

FIGS. 7A and 7B are views illustrating an arrangement of a sensor module of an electronic device according to an embodiment. Certain embodiments may include partition walls 730 between the light emitting units and light receiving units, or surrounding the light emitting units.

Referring to FIGS. 7A and 7B, the sensor module 700 may include a plurality of light emitting units 710 and a plurality of light receiving units 720. At least one of the separation distances between the plurality of light emitting units 710 and the plurality of light receiving units 720 may be different from the remaining separation distances. Partition walls 730 may be disposed between the light emitting units 710 and the light receiving units 720 mounted on a printed circuit board 701. The partition walls 730 may prevent light output from the light emitting units 710 from being directly input to the light receiving units 720 without being reflected by a user's body. The partition walls 730 may be formed in various shapes depending on the purpose of the partition walls 730 and the structures of mechanical parts near the partition walls 730.

For example, as illustrated in FIG. 7A, the partition walls 730 may be formed in a line shape between a first light emitting unit 711 and a first light receiving unit 721, between the first light receiving unit 721 and a second light emitting unit 712, between the second light emitting unit 712 and a second light receiving unit 722, between the second light receiving unit 722 and a third light emitting unit 713, between the third light emitting unit 713 and a third light receiving unit 723, and between the third light receiving unit 723 and the first light emitting unit 711.

In another example, as illustrated in FIG. 7B, the partition walls 730 may be formed to surround the first light emitting unit 711, the second light emitting unit 712, and the third light emitting unit 713, respectively. In another example, the partition walls 730 may be formed to surround the first light receiving unit 721, the second light receiving unit 722, and the third light receiving unit 723, respectively. Meanwhile, although the partition walls 730 are formed in a quadrangular shape in FIG. 7B, the disclosure is not limited thereto. For example, the partition walls 730 may be formed in a polygonal, circular, or oval shape other than the quadrangular shape.

The partition walls 730 may be perpendicular to the printed circuit board 701. The partition walls 730 may prevent light from the light emitting units 710 from directly being received by the light receiving units 723.

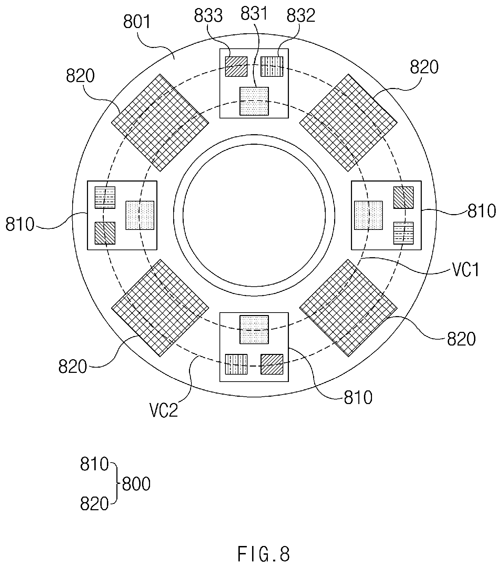

FIG. 8 is a view illustrating an arrangement of a sensor module of an electronic device according to an embodiment.

Referring to FIG. 8, the sensor module 800 may include a plurality of light emitting units 810 and a plurality of light receiving units 820 mounted on a printed circuit board 801. At least one of the separation distances between the plurality of light emitting units 810 and the plurality of light receiving units 820 may be different from the remaining separation distances.

The plurality of light emitting units 810 may be implemented to output light in various wavelength bands and/or light having various intensities. The plurality of light emitting units 810 may have the same type of emission wavelength or different types of emission wavelengths. Alternatively, at least one of the light emitting units 810 may have an emission wavelength of a different type from the emission wavelengths of the remaining light emitting units 810.

At least one of the light emitting units 810 may output light in a visible light band and/or an infrared band. At least one of the light emitting units 810 may include at least one light capable of outputting at least one of red light, green light, blue light, or infrared light. For example, the light emitting units 810 may include a first light body 831, a second light body 832, and a third light body 833. The first light body 831 may output green light having a wavelength of about 450 nm to about 650 nm, the second light body 832 may output red light having a wavelength of about 550 nm to about 700 nm, and the third light body 833 may output infrared light having a wavelength of about 880 nm to about 940 nm.

The first light body 831 may be physically spaced apart from each other with the light receiving units 820 therebetween. The first light body 831 may be disposed on the printed circuit board 801 along a virtual first ring VC1 at specified intervals. For example, the virtual first ring VC1 may be formed in a circular, oval, or polygonal shape.

The second light body 832 and the third light body 833 may be disposed adjacent to each other. The second light body 832 may be disposed on the printed circuit board 801 along a virtual second ring VC2 at specified intervals. The third light body 833 may be disposed on the printed circuit board 801 along the virtual second ring VC2 at specified intervals. The virtual second ring VC2 may be formed to surround the virtual first ring VC1. For example, the virtual second ring VC2 may be formed in a shape that is the same as, or different from, the shape of the virtual first ring VC1. The virtual second ring VC2 may be formed in a circular, oval, or polygonal shape.

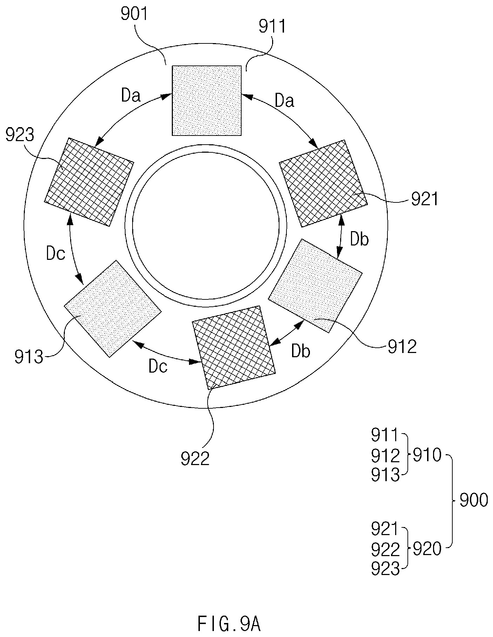

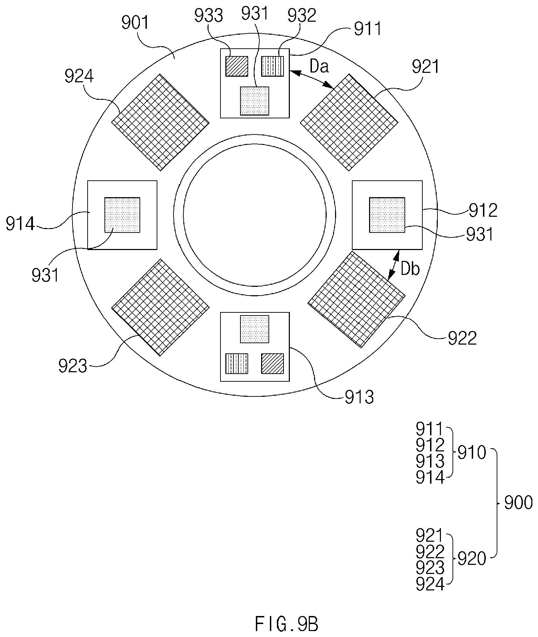

FIGS. 9A and 9B are views illustrating arrangements of sensor modules of electronic devices according to embodiments, and FIGS. 9C and 9D are views for explaining beam angles of light emitting units illustrated in FIGS. 9A and 9B.

Referring to FIGS. 9A and 9B, each of the sensor modules 900 may include a plurality of light emitting units 910 and a plurality of light receiving units 920.

According to an embodiment, as illustrated in FIG. 9A, the plurality of light emitting units 910 may include a first light emitting unit 911, a second light emitting unit 912, and a third light emitting unit 913. One of the first light emitting unit 911, the second light emitting unit 912, and the third light emitting unit 913 may have a beam angle different from that of at least one of the remaining light emitting units. Among the first light emitting unit 911, the second light emitting unit 912, and the third light emitting unit 913, the first light emitting unit 911 may have a wider beam angle than the second light emitting unit 912 and the third light emitting unit 913. The second light emitting unit 912 may have a narrower beam angle than the first light emitting unit 911 and the third light emitting unit 913. The third light emitting unit 913 may have a beam angle smaller than that of the first light emitting unit 911 and greater than that of the second light emitting unit 912. The plurality of light receiving units 920 may include a first light receiving unit 921, a second light receiving unit 922, and a third light receiving unit 923. The separation distances between the first to third light receiving units 921, 922, and 923 and the light emitting units 910 may be set depending on the beam angles of the adjacent light emitting units 910. The first light emitting unit 911 having a wider beam angle than the second light emitting unit 912 and the third light emitting unit 913 may be spaced apart from the first light receiving unit 921 and the third light receiving unit 923 by a first distance Da. The second light emitting unit 912 having a narrower beam angle than the first light emitting unit 911 and the third light emitting unit 913 may be spaced apart from the first light receiving unit 921 and the second light receiving unit 922 by a second distance Db shorter than the first distance Da. The third light emitting unit 913 having a beam angle narrower than that of the first light emitting unit 911 and wider than that of the second light emitting unit 912 may be spaced apart from the second light receiving unit 922 and the third light receiving unit 923 by a third distance Dc shorter than the first distance Da and longer than the second distance Db.

According to an embodiment, as illustrated in FIG. 9B, the plurality of light emitting units 910 may include a first light emitting unit 911, a second light emitting unit 912, a third light emitting unit 913, and a fourth light emitting unit 914. The plurality of light receiving units 920 may include a first light receiving unit 921, a second light receiving unit 922, a third light receiving unit 923, and a fourth light receiving unit 924. The sensor module 900 illustrated in FIG. 9B may include substantially the same components as the sensor module illustrated in FIG. 9A, except that the sensor module 900 illustrated in FIG. 9B further includes the fourth light emitting unit 914 and the fourth light receiving unit 924. Accordingly, the previous descriptions may be applied to the same components or operations.

Each of the first light emitting unit 911, the second light emitting unit 912, the third light emitting unit 913, and the fourth light emitting unit 914 illustrated in FIG. 9B may include a light that emits light in substantially the same wavelength band. For example, each of the first light emitting unit 911, the second light emitting unit 912, the third light emitting unit 913, and the fourth light emitting unit 914 may include a first light body 931 that emits light in substantially the same wavelength band. Each of the first light emitting unit 911 and the third light emitting unit 913 may include a second light body 932 and a third light body 933 that emit light in different wavelength bands. The second light body 932 and the third light body 933 may emit light in the same wavelength band, or may emit light in different wavelength bands.

According to an embodiment, the first light body 931 may output light in a green wavelength band that has a relatively high absorption coefficient. The absorption coefficient may refer to a coefficient indicating a rate at which output light emitted from the plurality of light emitting units 910 is reduced within a user's body. The light in the green wavelength band may be shallowly incident into the user's body tissue because the light in the green wavelength band has a relatively high absorption coefficient when compared to light in other wavelength bands. When the light in the green wavelength band is used, it is strong against motion, but the skin transmittance may be low.

One of the second light body 932 and the third light body 933 may output light in a red wavelength band that has a relatively low absorption coefficient, and the other one of the second light body 932 and the third light body 933 may output light in an infrared wavelength band that has a relatively low absorption coefficient. The light in the red wavelength band or the light in the infrared wavelength band may be deeply incident into the user's body tissue because the light in the red wavelength band or the light in the infrared wavelength band has a relatively low absorption coefficient. When the light in the red wavelength band or the light in the infrared wavelength band is used, the skin transmittance may be high. However, the signal strength may be weak, and the light may be sensitive to movement.

According to an embodiment, as illustrated in FIGS. 9C and 9D, light emitted from the plurality of light emitting units 910 may have various intensity ranges. As will be explained below, the angle between the rays with light intensity of 10% of the maximum intensity may be referred to the as the field angle θf. The angle between the rays with 50% intensity may be referred to as the beam angle θb1 or θb2.

Light emitted in a direction perpendicular to a light emitting surface of each of the plurality of light emitting units 910 may have a maximum luminous intensity (about 100%). An irradiation range of light representing an intensity of about 50% or more of the maximum luminous intensity (about 100%) may be referred to as the beam angle θb1 or θb2. The beam angle θb1 or θb2 may represent a range in which light from the light emitting units 910 is emitted. The beam angle θb1 or θb2 may be a characteristic value for determining the angle to which irradiation is possible at the position of maximum luminous intensity (about 100%). An irradiation range of light representing an intensity of about 10% or more of the maximum luminous intensity (about 100%) may be referred to as a field angle θf.

One light among the plurality of light emitting units 910 may have a beam angle different from that of at least one of the remaining light emitting units. For example, as illustrated in FIGS. 9C and 9D, the beam angle θb2 of the second light emitting unit 912 may be smaller than the beam angle θb1 of the first light emitting unit 911. The separation distance between the second light emitting unit 912 having the small beam angle θb2 and the second light receiving unit 922 may be shorter than the separation distance between the first light emitting unit 911 having the large beam angle θb1 and the first light receiving unit 921.

In certain embodiments, the distance between the light emitting units and the adjacent light receiving unit may be based on the beam angle θb1. For example, the distance between the light receiving unit and the light emitting unit can be set such that a reflection of light at the beam angle at a predetermined distance will be received by the light receiving unit. For example, the distance between the light receiving unit and the light emitting unit can be set such that a ray of light emitted from the light emitting unit at the beam angle that is reflected from the user's skin at a 2mm distance will be received by the light receiving unit. In certain embodiments, the distance between the light receiving unit and the light emitting unit can be several mm (e.g.1mm to 5mm) to several tens of mm , depending on the typical distance between the sensor module and the user's skin.

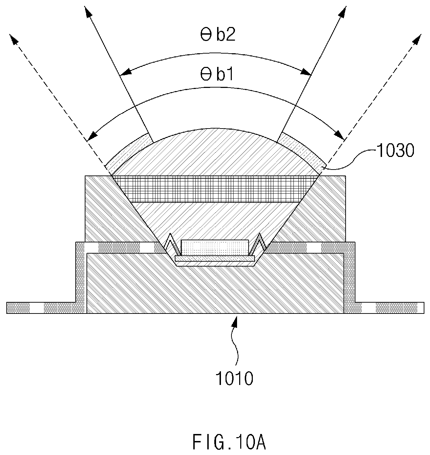

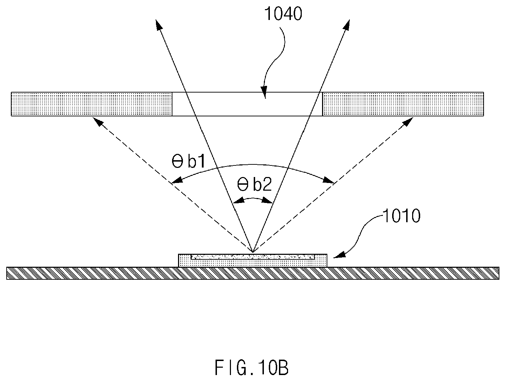

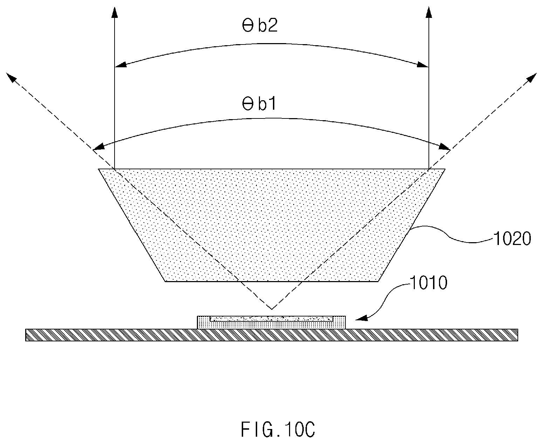

FIGS. 10A, 10B, and 10C are views illustrating various examples of a light according to an embodiment.

Referring to FIGS. 10A, 10B, and 10C, the beam angle of the light 1010 may be adjusted through an optical member disposed over a light emitting chip or a component (e.g., an electrode or/and an insulator) included in the light emitting chip. The beam angle may be reduced by, for example, light blocking film (FIG. 10A), a slit that limits the emissive region, (FIG. 10B), or lens structure (FIG. 10C)..

For example, as illustrated in FIG. 10A, an optical member formed of a light blocking film 1030 may be disposed over the light emitting chip of the light 1010. The emissive region of light emitted from the light emitting chip at the first beam angle θb1 may be narrowed by the light blocking film 1030, and therefore the first beam angle θb1 of the light 1010 may be reduced to a second beam angle θb2. The light 1010 that does not include the light blocking film 1030 may emit light at the first beam angle θb1. The light 1010 including the light blocking film 1030 may emit light at the second beam angle θb2 smaller than the first beam angle θb1.

In another example, as illustrated in FIG. 10B, an optical member including a slit 1040 may be disposed over the light emitting chip of the light 1010. A portion of light emitted from the light emitting chip at the first beam angle θb1 may be output through the slit 1040, and the remaining portion of the light may be blocked by a region other than the slit 1040. Accordingly, the emissive region of the light emitted from the light emitting chip at the first beam angle θb1 may be limited to the area of the slit 1040, and therefore the first beam angle θb1 of the light 1010 may be reduced to the second beam angle θb2. The light 1010 that does not include the slit 1040 may emit light at the first beam angle θb1. The light 1010 including the slit 1040 may emit light at the second beam angle θb2 smaller than the first beam angle θb1.

In another example, as illustrated in FIG. 10C, an optical member formed of a lens structure 1020 may be disposed over the light emitting chip of the light 1010. The emissive region of light emitted from the light emitting chip at the first beam angle θb1 may be narrowed by the lens structure 1020, and therefore the first beam angle θb1 of the light 1010 may be reduced to the second beam angle θb2. For example, the lens structure 1020 may include at least one of a total internal reflection (TIR) lens or a ball lens. The light 1010 that does not include the lens structure 1020 may emit light at the first beam angle θb1. The light 1010 including the lens structure 1020 may emit light at the second beam angle θb2 smaller than the first beam angle θb1.

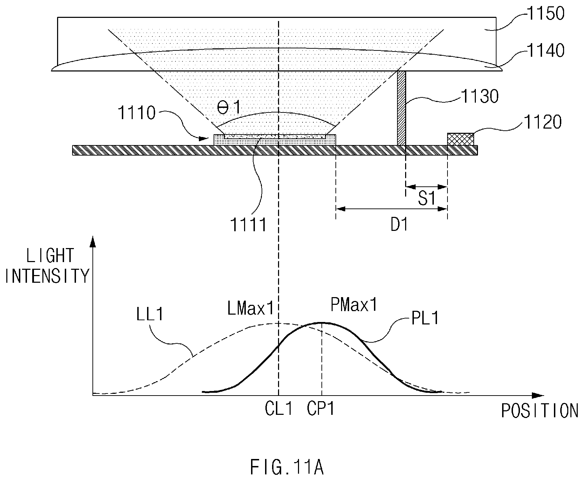

FIGS. 11A to 11C are views illustrating examples of measuring biometric signals using an electronic device including a sensor module according to an embodiment.

Referring to FIGS. 11A to 11C, the electronic device according to an embodiment may include a window 1140, a light 1110, a light receiving unit 1120, and a partition wall 1130.

One side of the window 1140 (e.g., the front plate 201 of FIGS. 2 and 3) may make contact with a body 1150 of a user, and an opposite side of the window 1140 may make contact with the partition wall 1130.

The partition wall 1130 may prevent light emitted from at least one light 1111 included in the light 1110 from being directly input to the light receiving unit 1120. The partition wall 1130 may be formed of a light absorbing material to prevent light in any wavelength band from being directly input to the light receiving unit 1120. The partition wall 1130 may be disposed between the light 1110 and the light receiving unit 1120. The separation distance between the light 1110 and the partition wall 1130 may be equal to, or different from, the separation distance between the light receiving unit 1120 and the partition wall 1130.

The light 1110 may include the at least one light 1111. When the light 1111 applies light to the body 1150 of the user, the light may be diffused within the body 1150 of the user. Most of the diffused light may be absorbed into the user's skin, blood, and tissue, and a portion of the light may be reflected and radiated out of the skin in the process of diffusion and scattering. A portion of the light radiated out of the skin may be obtained through the light receiving unit 1120.

According to an embodiment, it may be difficult to obtain, through the light receiving unit 1120, light travelling toward the opposite side to the light receiving unit 1120 after input to the user's skin. Light travelling toward the light receiving unit 1120 after input to the user's skin may be obtained through the light receiving unit 1120. Light incident to the user's skin so as to be close to the light receiving unit 1120 may be obtained through the light receiving unit 1120. Accordingly, virtual centers CP1, CP2, and CP3 where the maximum luminous intensities PMax1, PMax2, PMax3 of reflected light beams PL1, PL2, PL3 incident to the light receiving unit 1120 appear may be biased toward the light receiving unit 1120 when compared to virtual centers CL1, CL2, and CL3 where the maximum luminous intensities LMax1, LMax2, and LMax3 of output light beams LL1, LL2, and LL3 emitted through the light 1110 appear. The light receiving unit 1120 may receive the reflected light beams PL1, PL2, and PL3 having high light intensities only when the light receiving unit 1120 is disposed close to the virtual centers CP1, CP2, and CP3 of the reflected light beams PL1, PL2, and PL3 incident to the light receiving unit 1120. As the beam angles θ1, θ2, and θ3 of the light are decreased, the virtual centers CP1, CP2, and CP3 of the reflected light beams PL1, PL2, and PL3 may move toward the virtual centers CL1, CL2, and CL3 of the output light beams LL1, LL2, and LL3, and as the beam angles θ1, θ2, and θ3 of the light 1110 are increased, the virtual centers CP1, CP2, and CP3 of the reflected light beams PL1, PL2, and PL3 may move away from the virtual centers CL1, CL2, and CL3 of the output light beams LL1, LL2, and LL3.

The separation distance between the light 1110 and the light receiving unit 1120 may be set in consideration of the beam angle of the light 1110. As the beam angle of the light 1110 is decreased, the separation distance between the light 1110 and the light receiving unit 1120 may be set to be relatively short. As the beam angle of the light 1110 is increased, the separation distance between the light 1110 and the light receiving unit 1120 may be set to be relatively long. As the beam angle of the light 1110 is decreased, the virtual centers CP1, CP2, and CP3 of the reflected light beams PL1, PL2, and PL3 incident to the light receiving unit 1120 may move toward the light receiving unit 1120. Accordingly, the light receiving unit 1120 may receive the reflected light beams PL1, PL2, and PL3 with low light loss.

For example, as illustrated in FIG. 11A, the light 1110 may have the relatively wide first beam angle θ1. In this case, the light 1110 and the light receiving unit 1120 may be spaced apart from each other by a first distance D1. At least one of the light 1110 or the light receiving unit 1120 may be spaced apart from the partition wall 1130 by a first separation distance S1. The first output light beam LL1 emitted through the light 1110 having the first beam angle θ1 may be reflected from the body 1150 of the user. The reflected first output light beam LL1 may be obtained as the first reflected light beam PL1 with low light loss through the light receiving unit 1120 spaced apart from the light 1110 by the first distance D1.

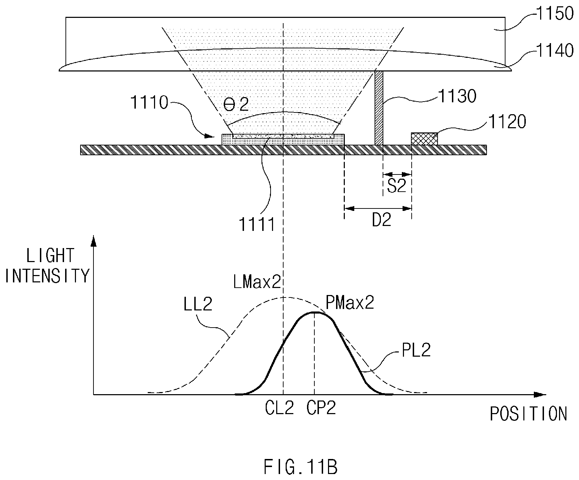

In another example, as illustrated in FIG. 11B, the light 1110 may have the second beam angle θ2 narrower than the first beam angle θ1. In this case, the light 1110 and the light receiving unit 1120 may be spaced apart from each other by a second distance D2 shorter than the first distance D1. At least one of the light 1110 or the light receiving unit 1120 may be spaced apart from the partition wall 1130 by a second separation distance S2 shorter than the first separation distance S1. The second output light beam LL2 emitted through the light 1110 having the second beam angle θ2 narrower than the first beam angle θ1 may be reflected from the body 1150 of the user. The reflected second output light beam LL2 may be obtained as the second reflected light beam PL2 with low light loss through the light receiving unit 1120 spaced apart from the light 1110 by the second distance D2 shorter than the first distance D1.

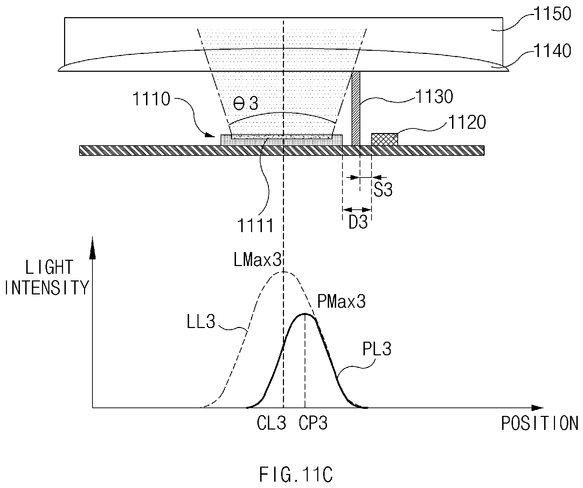

In another example, as illustrated in FIG. 11C, the light 1110 may have the third beam angle θ3 narrower than the second beam angle θ2. In this case, the light 1110 and the light receiving unit 1120 may be spaced apart from each other by a third distance D3 shorter than the second distance D2. At least one of the light 1110 or the light receiving unit 1120 may be spaced apart from the partition wall 1130 by a third separation distance S3 shorter than the second separation distance S2. Accordingly, the third output light beam LL3 emitted through the light 1110 having the third beam angle θ3 narrower than the second beam angle θ2 may be reflected from the body 1150 of the user. The reflected third output light beam LL3 may be obtained as the third reflected light beam PL3 with high light intensity through the light receiving unit 1120 spaced apart from the light 1110 by the third distance D3 shorter than the second distance D2.

According to an embodiment, the reflected light beams PL1, PL2, and PL3 sensed through the light receiving unit 1120 may include a direct current (DC) component and an alternating current (AC) component. For example, the reflected light beams PL1, PL2, and PL3 may include a direct current (DC) component having a certain magnitude that returns from skin, tissue, or pigment from which light emitted from the light 1110 is reflected and an AC component having a certain magnitude that returns from the user's blood from which the light emitted from the light 1110 is reflected. The AC component may have amplitude and period due to a blood change caused by a heart beat occurring depending on heart movement. For example, the reflected light beams PL1, PL2, and PL3 may include at least one of a venous blood DC component by absorption or reflection of venous blood in a capillary, an arterial blood DC component by absorption or reflection of arterial blood in a capillary, or an AC component by arterial blood.

According to an embodiment, the proportions of components of reflected light obtained by the light receiving unit 1120 may vary depending on the beam angle of the light 1110. For example, as illustrated in FIG. 11A, the light 1110 having the large beam angle θ1 may be disposed at a long separation distance from the light receiving unit 1120. The first output light beam LL1 emitted from the light 1110 having the large beam angle θ1 may be reflected after passing through a blood vessel inside the body 1150 of the user and then may be incident to the light receiving unit 1120 as the first reflected light beam PL1. Accordingly, the proportion of the AC component of the first reflected light beam PL1 may be higher than the proportion of the DC component of the first reflected light beam PL1.

As illustrated in FIG. 11C, the light 1110 having the small beam angle θ3 may be disposed at a short separation distance from the light receiving unit 1120. The third output light beam LL3 emitted from the light 1110 having the small beam angle θ3 may be reflected from the skin tissue without passing through the user's blood vessel and then may be incident to the light receiving unit 1120 as the third reflected light beam PL3. Accordingly, the proportion of the DC component of the third reflected light beam PL3 may be higher than the proportion of the AC component of the third reflected light beam PL3. Although the proportion of the AC component of the third reflected light beam PL3 is lower than the proportion of the DC component of the third reflected light beam PL3, the magnitude of the AC component of the third reflected light beam PL3 may be greater than the magnitude of the proportion of the DC component of the third reflected light beam PL3.

According to an embodiment, the magnitude of an AC component of reflected light obtained by the light receiving unit 1120 may vary depending on the separation distance between the light 1110 and the light receiving unit 1120 that is determined by the beam angles θ1, θ2, and θ3 of the light 1110. The magnitude of the AC component of the third reflected light beam PL3 obtained through the light receiving unit 1120 disposed at the short separation distance from the light 1110 may be greater than the magnitude of the AC component of the first reflected light beam PL1 obtained through the light receiving unit 1120 disposed at the long separation distance from the light 1110. Because the AC component of the third reflected light beam PL3 obtained through the light receiving unit 1120 disposed at the short separation distance from the light 1110 having the small beam angle has a relatively large magnitude, the light receiving unit 1120 may obtain sufficient light receiving efficiency even when the amount of light of the light 1110 is reduced. Accordingly, signal quality performance may be maintained even though the intensity of current supplied to the light 1110 having the small beam angle is lowered. In addition, current consumption may be minimized because the intensity of the current supplied to the light 1110 having the small beam angle is able to be lowered.

According to an embodiment, the range of light applied to the user's skin may vary depending on the beam angle of the light 1110. As the beam angle of the light 1110 is increased, the irradiation range of output light incident into the user's skin may be widened. Because the irradiation range of the output light is widened, the reflection range of reflected light obtained through the light receiving unit 1120 may also be widened, and thus a reflected light signal may be easy to measure. For example, as illustrated in FIG. 11A, the first reflected light beam PL1 obtained through the light receiving unit 1120 disposed at the long separation distance from the light 1110 having the large beam angle may have a relatively wide light distribution range. The amplitude of the AC component of the first reflected light beam PL1 may be relatively small. Accordingly, it may be easy to remove motion artifacts included in the AC component of the first reflected light bam PL1, and an influence of the motion artifacts may be reduced.

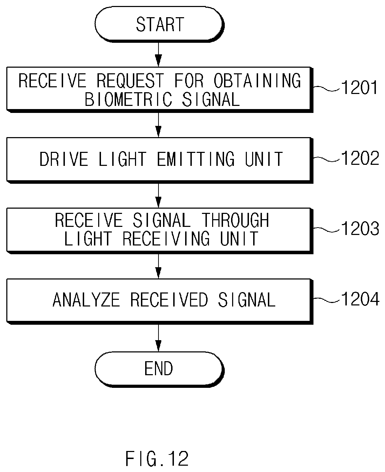

FIG. 12 is a flowchart for explaining a method of identifying signal characteristics of a sensor module of an electronic device according to an embodiment.

In the following embodiment, operations may be sequentially performed. However, the operations are not necessarily sequentially performed. For example, the sequence of the operations may be changed, or at least two operations may be performed in parallel.

Operations of the electronic device (e.g., the electronic device 200 of FIGS. 2A and 2B, the electronic device 300 of FIG. 3, or the biometric signal processing device 401 of FIG. 4) including the sensor module according to the embodiment illustrated in FIG. 12 (e.g., the sensor module 176 of FIG. 1, the sensor module 410 of FIG. 4, the sensor module 500 of FIG. 5, the sensor module 600 of FIG. 6, the sensor module 700 of FIGS. 7A and 7B, the sensor module 800 of FIG. 8, or the sensor module 900 of FIGS. 9A and 9B) may be performed by a processor (e.g., the processor 120 of FIG. 1 or the processor 420 of FIG. 4). The following description will be given with reference to the biometric signal processing device 401 of FIG. 4 and the structure of the sensor module 900 of FIG. 9B. Meanwhile, description of the technical features described above will hereinafter be omitted.

Referring to FIG. 12, in operation 1201, the processor 420 may identify reception of a request for obtaining a biometric signal corresponding to a body. The biometric signal corresponding to the body may be related to at least one of heart rate, stress index, blood oxygen saturation, maximum heart rate, body fat, local body fat, skin tone, melanin, wrinkle, skin moisture level, blood sugar, or blood pressure.

The request for obtaining the biometric signal corresponding to the body may be received based on a user command or a pre-specified schedule. According to an embodiment, the request for obtaining the biometric signal may be received based on the occurrence of an event previously set by a user or an event depending on the user's health condition.

For example, the case based on the user's command may include a case in which the user executes an application related to the acquisition of the biometric signal. In another example, the case based on the user's command may include a case of receiving a user input for activating the sensor module 900 including the light 910 and the light receiving unit 920. In another example, the case based on the user's command may include a case of receiving a request for a user biometric signal from an external device through a communication module.

In operation 1202, when receiving the request for obtaining the biometric signal, the processor 420 may drive at least one of the plurality of light emitting units 910 included in the sensor module 900. The processor 420 may drive the plurality of light emitting units 910 for which the separation distances from the plurality of light receiving units 920 are set depending on the beam angles. Depending on the beam angles of the plurality of light emitting units 910, at least one of the separation distances between the plurality of light emitting units 910 and the plurality of light receiving units 920 may be different from the remaining separation distances.

For example, the processor 420 may drive the plurality of light emitting units 910 at different timings. For example, the processor 420 may perform control such that the first light emitting unit 911, the second light emitting unit 912, the third light emitting unit 913, and the fourth light emitting unit 914 sequentially emit light.

In another example, the processor 420 may drive the plurality of light emitting units 910 at the same timing. For example, the processor 420 may perform control such that the first light emitting unit 911, the second light emitting unit 912, the third light emitting unit 913, and the fourth light emitting unit 914 simultaneously emit light.

According to an embodiment, the processor 420 may determine at least one of the intensity of light or the irradiation range of the plurality of light emitting units 910, based on user-related biometric information stored in the memory 430. The memory 430 may store at least one of the skin tone, the blood pressure, the blood glucose level, the heart rate, the blood oxygen saturation, the stress index, or the body fat index of the user. For example, the processor 420 may determine at least one of the intensity of light or the irradiation range of the plurality of light emitting units 910, based on the user's skin tone stored in the memory 430. When the user's skin tone is relatively light, the processor 420 may control the plurality of light emitting units 910 to output light having a relatively lower intensity than when the user's skin tone is relatively dark. When the user's skin tone is relatively dark, the processor 420 may control the plurality of light emitting units 910 to output light having a relatively higher intensity than when the user's skin tone is relatively light. In another example, the processor 420 may select the light 910 and the light receiving unit 920 that have a separation distance at which sensing performance for biometric characteristics is optimal in the structure and position of the user's capillary stored in the memory 430 and may control driving of the selected light 910.

In operation 1203, the processor 420 may perform control such that at least one of the light receiving units 920 obtains light output through at least one of the light emitting units 910.

For example, in operation 1203, the processor 420 may perform control such that the plurality of light receiving units 920 sequentially receive light signals as the plurality of light emitting units 910 are sequentially driven in operation 1202. For example, the processor 420 may receive light signals sequentially detected through the first light receiving unit 921, the second light receiving unit 922, the third light receiving unit 923, and the fourth light receiving unit 924 after light beams sequentially emitted through the first light emitting unit 911, the second light emitting unit 912, the third light emitting unit 913, and the fourth light emitting unit 914 are reflected from the user's body.

For example, in operation 1203, the processor 420 may perform control such that the plurality of light receiving units 920 simultaneously receive light signals as the plurality of light emitting units 910 are simultaneously driven in operation 1202. For example, the processor 420 may receive light signals simultaneously detected through the first light receiving unit 921, the second light receiving unit 922, the third light receiving unit 923, and the fourth light receiving unit 924 after light beams simultaneously emitted through the first light emitting unit 911, the second light emitting unit 912, the third light emitting unit 913, and the fourth light emitting unit 914 are reflected from the user's body.

In operation 1204, the processor 420 may sense a biometric signal based on at least some of the sensed light signals. The processor 420 may sense a biometric signal by analyzing at least one of DC components or AC components of the light signals sensed through the first light receiving unit 921, the second light receiving unit 922, the third light receiving unit 923, and the fourth light receiving unit 924.

For example, the processor 420 may determine whether the electronic device is worn on the user's body, by analyzing the DC components of the light signals sensed through the plurality of light receiving units 920. When a DC component of a light signal sensed through at least one of the first light receiving unit 921, the second light receiving unit 922, the third light receiving unit 923, or the fourth light receiving unit 924 is greater than or equal to a specified value, the processor 420 may determine that the electronic device is worn on the user's body. When the DC component of the light signal sensed through at least one of the first light receiving unit 921, the second light receiving unit 922, the third light receiving unit 923, or the fourth light receiving unit 924 is less than the specified value, the processor 420 may determine that the electronic device is not worn on the user's body. For example, when the magnitude of a DC component of a light signal in a specified wavelength band is greater than or equal to a specified value, the processor 420 may determine that the electronic device is worn on the user's body. The processor 420 may determine whether the electronic device is worn on the user's body, based on a DC component of an infrared light signal that is least affected by melanin existing in the user's skin

In another example, the processor 420 may estimate a biometric signal including the user's heart rate, blood glucose level, or blood pressure by analyzing the AC components of the light signals sensed through the plurality of light receiving units 920.

In another example, the processor 420 may estimate a biometric signal including the user's oxygen saturation by analyzing the AC components and the DC components of the light signals sensed through the plurality of light receiving units 920. The processor 420 may estimate the oxygen saturation through the ratio between the magnitudes of the AC components and the magnitudes of the DC components of the light signals sensed through the plurality of light receiving units 920. For example, the processor 420 may measure the user's oxygen saturation based on the ratio between the magnitudes of AC components and the magnitudes of DC components of red light signals sensed through the plurality of light receiving units 920 and the ratio between the magnitudes of AC components and the magnitudes of DC components of infrared light signals sensed through the plurality of light receiving units 920.

According to an embodiment, the processor 420 may determine the user's biometric characteristics using at least one of a plurality of biometric signals. The processor 420 may compare characteristics of the plurality of biometric signals and may determine an optimal biometric signal for acquisition of biometric information based on the comparison result. The processor 420 may determine the user's biometric characteristics by analyzing a signal having a high frequency strength or AC component among the plurality of biometric signals as a main signal. The processor 420 may determine the user's biometric characteristics by analyzing the remaining signals other than the main signal among the plurality of biometric signals as auxiliary signals. The processor 420 may determine the user's biometric characteristics by averaging the auxiliary signals.

For example, the processor 420 may determine the user's biometric characteristics by analyzing a biometric signal sensed through the light receiving unit 920 having the shortest separation distance from the light emitting units 910 among the plurality of light receiving units 920 as the main signal. The processor 420 may determine the user's biometric characteristics by analyzing biometric signals sensed through the remaining light receiving units as auxiliary signals.

According to an embodiment, the processor 420 may compare a plurality of biometric signals obtained through the plurality of light receiving units 920 and may select a biometric signal determined to be less affected by noise or may correct a signal based on identified noise. The noise may include at least one of motion artifacts or ambient noise. For example, when one of the plurality of biometric signals experiences a rapid change in a specific section, it may be determined that a noise component is included in the specific section, and in the specific section, a biometric signal may be obtained based on another biometric signal among the plurality of biometric signals. In another example, the processor 420 may obtain an accurate biometric signal by analyzing noise from one of the plurality of biometric signals and compensating for noise analyzed from another biometric signal among the plurality of biometric signals.

The processor 420 may convert the sensed biometric signal into biometric information through a predetermined algorithm. The converted biometric information may be displayed through a user interface, or may be transferred to an external device.

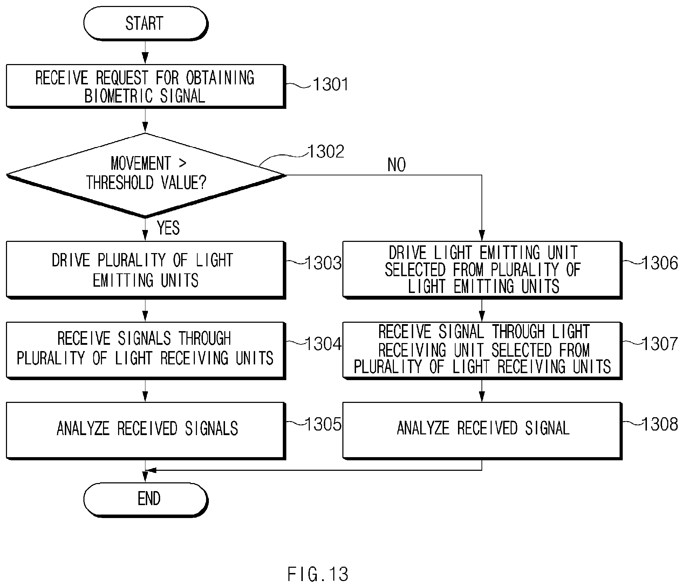

FIG. 13 is a flowchart for explaining a method of identifying signal characteristics of a sensor module of an electronic device according to an embodiment.

In the following embodiment, operations may be sequentially performed. However, the operations are not necessarily sequentially performed. For example, the sequence of the operations may be changed, or at least two operations may be performed in parallel.

Operations of the electronic device (e.g., the electronic device 200 of FIGS. 2A and 2B, the electronic device 300 of FIG. 3, or the biometric signal processing device 401 of FIG. 4) including the sensor module according to the embodiment illustrated in FIG. 13 (e.g., the sensor module 176 of FIG. 1, the sensor module 410 of FIG. 4, the sensor module 500 of FIG. 5, the sensor module 600 of FIG. 6, the sensor module 700 of FIGS. 7A and 7B, the sensor module 800 of FIG. 8, or the sensor module 900 of FIGS. 9A and 9B) may be performed by a processor (e.g., the processor 120 of FIG. 1 or the processor 420 of FIG. 4). The following description will be given with reference to the biometric signal processing device 401 (or, the electronic device) of FIG. 4 and the structure of the sensor module 900 of FIG. 9B. Meanwhile, description of the technical features described above will hereinafter be omitted.

Referring to FIG. 13, in operation 1301, the processor 420 may identify reception of a request for obtaining a biometric signal corresponding to a body. For example, the processor 420 may perform an operation substantially the same as operation 1201 of FIG. 12.

In operation 1302, the processor 420 may sense movement of the electronic device 401 and may determine a current state of a user wearing the electronic device 401 (e.g., a normal state, a sleep state, an exercise state, or a state in which a specified event occurs), based on the degree of the sensed movement. For example, the processor 420 may determine the current state of the user wearing the electronic device 401, based on a motion signal of a motion sensor included in the sensor module. The motion sensor may be electrically connected with the processor 420 and may provide a motion signal generated depending on the detection of movement of the electronic device 401 to the processor 420.

Based on the motion signal of the motion sensor, the processor 420 may determine an activity state (or, a state in which the user makes a big movement) (e.g., an exercise state) when the movement of the electronic device 401 exceeds a specified threshold value. Based on the motion signal of the motion sensor, the processor 420 may determine an inactivity state (or, a state in which the user makes a little movement or makes no movement) (e.g., a stationary state, a sedentary state, or a sleep state) when the movement of the electronic device 401 is less than or equal to the specified threshold value. In certain embodiments, a big movement of the activity state can be a movement in excess of a predetermined distance (e.g. 5cm). In certain embodiments, a small movement of the inactivity state can be movement less than the predetermined distance (e.g.5cm).

Meanwhile, although it has been described that the current state of the user is sensed through the motion sensor, the disclosure is not limited thereto. For example, the current state of the user may be identified based on biometric information (e.g., heart rate information) from light signals received through the plurality of light receiving units 920. In another example, the current state of the user may be identified based on the motion signal from the motion sensor and the biometric information (e.g., heart rate information) from the plurality of light receiving units 920.

When the processor 420, in operation 1302, determines that the user makes a big movement (YES in operation 1302), the processor 420 may, in operation 1303, drive at least one of the plurality of light emitting units 910 included in the sensor module 900. For example, in operation 1303, the processor 420 may perform an operation substantially the same as operation 1202.

In operation 1304, the processor 420 may perform control such that at least one of the light receiving units obtains light output through at least one of the light emitting units. For example, in operation 1304, the processor 420 may perform an operation substantially the same as operation 1203.

In operation 1305, the processor 420 may sense a biometric signal based at least partly on the sensed light signal. For example, in operation 1305, the processor 420 may perform an operation substantially the same as operation 1204.

When the processor 420, in operation 1302, determines that the user makes a little movement (NO in operation 1302), the processor 420 may, in operation 1306, perform control to drive a light 910 selected from the plurality of light emitting units 910 included in the sensor module 900. For example, the processor 420 may perform control to drive (e.g., activate or turn on) a light 910 having a relatively small beam angle among the plurality of light emitting units 910. The processor 420 may perform control so as not to drive a light 910 having a relatively large beam angle among the plurality of light emitting units 910. For example, the processor 420 may perform control such that among the first to fourth light emitting units 911, 912, 913, and 914, the second light emitting unit 912 having a relatively small beam angle is driven and the first light emitting unit 911, the third light emitting unit 913, and the fourth light emitting unit 914 that have relatively large beam angles are not driven.

The processor 420 may perform control such that the plurality of light emitting units 910 output light having a specified range of output intensity. The processor 420 may perform control such that the second light 912 having a relatively small beam angle outputs light having low intensity when compared to the first light emitting unit 911, the third light emitting unit 913, and the fourth light emitting unit 914 that have relatively large beam angles. The processor 420 may perform control such that the second light emitting unit 912 having a relatively small beam angle outputs light having lower intensity when the user makes a little movement than when the user makes a big movement.

In operation 1307, the processor 420 may perform control to drive a light receiving unit 920 selected from the plurality of light receiving units. For example, the processor 420 may perform control to drive a light receiving unit 920 disposed at a short distance from a light 910 having a relatively small beam angle among the plurality of light receiving units 920. The processor 420 may perform control so as not to drive a light receiving unit 920 disposed at a long distance from a light 910 having a relatively large beam angle among the plurality of light receiving units 920. For example, the processor 420 may perform control to drive the second light receiving unit 922 disposed at a short distance from the second light 912 having a relatively small beam angle among the first to fourth light receiving units 921, 922, 923, and 924. The processor 420 may perform control so as not to drive the first, third, and fourth light receiving units 921, 923, and 924 disposed at long distances from the first, third, and fourth light emitting units 911, 913, and 914 that have relatively large beam angles.

The processor 420 may perform control such that among the plurality of light receiving units, a light receiving unit 920 disposed at a short distance from the light emitting units 910 obtains light output through a light 910 having a relatively small beam angle.

In operation 1308, the processor 420 may sense a biometric signal based on at least a portion of the light signal obtained through the light receiving unit 920 disposed at the short distance from the light emitting units 910 among the plurality of light receiving units 920. For example, the processor 420 may sense a biometric signal by analyzing at least one of a DC component or an AC component of a light signal sensed through the second light receiving unit 922 disposed at a relatively short distance from the second light 912 having a relatively small beam angle.

The processor 420 may convert the sensed biometric signal into biometric information through a predetermined algorithm. The converted biometric information may be displayed through a user interface, or may be transferred to an external device.

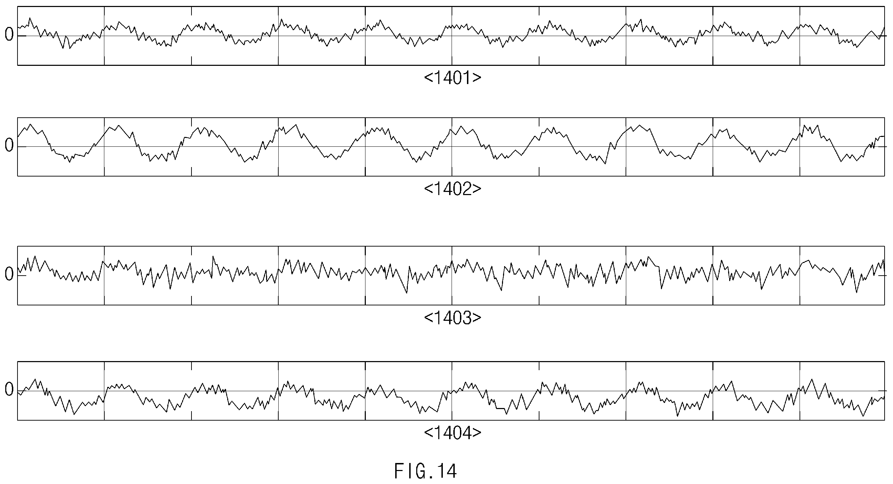

FIG. 14 illustrates biometric signals obtained through an electronic device according to an embodiment.

Referring to FIG. 14, the electronic device according to an embodiment may obtain a plurality of biometric signals through an optical sensor including a plurality of light emitting units and a plurality of light receiving units. For example, a first biometric signal 1401 may be a biometric signal obtained by driving a first light emitting unit (e.g., the first light 911 emitting unit of FIG. 9B) and removing noise from a light signal received through a first light receiving unit (e.g., the first light receiving unit 921 of FIG. 9B). A second biometric signal 1402 may be a biometric signal obtained by driving a second light emitting unit (e.g., the second light emitting unit 912 of FIG. 9B) and removing noise from a light signal received through a second light receiving unit (e.g., the second light receiving unit 922 of FIG. 9B). A third biometric signal 1403 may be a biometric signal obtained by driving a third light emitting unit (e.g., the third light emitting unit 913 of FIG. 9B) and removing noise from a light signal received through a third light receiving unit (e.g., the third light receiving unit 923 of FIG. 9B). A fourth biometric signal 1404 may be a biometric signal obtained by driving a fourth light emitting unit (e.g., the fourth light emitting unit 914 of FIG. 9B) and removing noise from a light signal received through a fourth light receiving unit (e.g., the fourth light receiving unit 924 of FIG. 9B).

A processor (e.g., the processor 420 of FIG. 4) may determine a user's biometric characteristics using at least one of the plurality of biometric signals. The processor may compare characteristics of the plurality of biometric signals and may determine an optimal biometric signal for acquisition of biometric information based on the comparison result. For example, the processor may compare each biometric signal with the magnitude of at least one of noise (e.g., signal to noise ratio (SNR)) or an AC component (e.g., a peak-to-peak value) and may determine an optimal biometric signal for acquisition of biometric information, based on the comparison result.

For example, the processor may determine the user's biometric characteristics using a signal having a high AC component among the plurality of biometric signals. Because an AC component of the second biometric signal 1402 illustrated in FIG. 14 is greater than those of the first biometric signal 1401, the third biometric signal 1403, and the fourth biometric signal 1404, the processor may determine the user's biometric characteristics by analyzing the second biometric signal 1402 as a main signal. The processor may determine the user's biometric characteristics by analyzing the first biometric signal 1401, the third first biometric signal 1403, and the fourth first biometric signal 1404 as auxiliary signals. The processor may determine the user's biometric characteristics by averaging the first biometric signal 1401, the third first biometric signal 1403, and the fourth first biometric signal 1404.

The above-described electronic devices are not limited to the embodiments described with reference to the drawings, and the embodiments described with reference to the drawings may be applied in combination.

The accuracy and quality of a biometric signal may be degraded depending on a body structure of a user wearing an electronic device and a current state of the user.

Embodiment of the disclosure described herein relate to an electronic device for measuring a biometric signal suitable for a body structure and a current state of a user.

The technical problems to be solved by the disclosure are not limited to the aforementioned problems, and any other technical problems not mentioned herein will be clearly understood from the following description by those skilled in the art to which the disclosure pertains.

According to an embodiment of the disclosure, an electronic device may include a plurality of light emitting units spaced apart from each other to form a ring shape on a printed circuit board, at least one light receiving unit disposed between the plurality of light emitting units, and a processor that analyzes light generated from the light emitting units and incident to the light receiving unit, and a separation distance between each of the plurality of light emitting units and the at least one light receiving unit such that the at least one light receiving unit is configured to receive reflected light from light emitted at a beam angle from each of the plurality of light emitting units and reflected from a predetermined distance from the printed circuit board.

According to an embodiment, the at least one light receiving unit may include a plurality of light receiving units, and at least one separation distance among separation distances between the plurality of light emitting units and the plurality of light receiving units may be different from the remaining separation distances.

According to an embodiment, the plurality of light emitting units may include a first light emitting unit and a second light emitting unit having different beam angles, and the at least one light receiving unit may include a first light receiving unit spaced apart from the first light emitting unit by a first distance and spaced apart from the second light emitting unit by a second distance different from the first distance.

According to an embodiment, the plurality of light emitting units may include a first light emitting unit, a second light emitting unit, and a third light emitting unit having different beam angles. The at least one light receiving unit may include a first light receiving unit, a second light receiving unit, and a third light receiving unit. The first light emitting unit may be disposed between the first light receiving unit and the third light receiving unit and may be spaced apart from the first light receiving unit and the third light receiving unit by a first distance. The second light emitting unit may be disposed between the first light receiving unit and the second light receiving unit and may be spaced apart from the first light receiving unit and the second light receiving unit by a second distance different from the first distance. The third light emitting unit may be disposed between the second light receiving unit and the third light receiving unit and may be spaced apart from the second light receiving unit and the third light receiving unit by a third distance different from the first distance and the second distance.

According to an embodiment, the plurality of light emitting units may include a first light emitting unit and a second light emitting unit having different beam angles. The at least one light receiving unit may include a first light receiving unit, a second light receiving unit, a third light receiving unit, and a fourth light receiving unit. The first light emitting unit may be disposed between the first light receiving unit and the second light receiving unit and may be spaced apart from the first light receiving unit and the second light receiving unit by a first distance. The second light emitting unit may be disposed between the third light receiving unit and the fourth light receiving unit and may be spaced apart from the third light receiving unit and the fourth light receiving unit by a second distance different from the first distance.

According to an embodiment, each of the first light emitting unit and the second light emitting unit may include at least one first light body that emits light in a specified wavelength band. The first light body of the first light emitting unit and the first light body of the second light emitting unit may have different beam angles.

According to an embodiment, the first light emitting unit may have a larger beam angle than the third light emitting unit, and the third light emitting unit may have a larger beam angle than the second light emitting unit. The first distance may be longer than the third distance, and the third distance may be shorter than the second distance.

According to an embodiment, the electronic device may further include a partition wall disposed between the first light emitting unit and the first light receiving unit, a partition wall disposed between the first light receiving unit and the second light emitting unit, a partition wall disposed between the second light emitting unit and the second light receiving unit, a partition wall disposed between the second light receiving unit and the third light emitting unit, a partition wall disposed between the third light emitting unit and the third light receiving unit, and a partition wall disposed between the third light receiving unit and the first light emitting unit.

According to an embodiment, the second light emitting unit may include a light emitting chip that generates light in the same wavelength band as the first light emitting unit and an optical member disposed over the light emitting chip. The optical member may be formed of a lens structure or an optical film that restricts an emission range of the light generated from the light emitting chip.

According to an embodiment, the second light emitting unit may include a light emitting chip that generates light in the same wavelength band as the first light emitting unit and has a smaller light emission range than the first light emitting unit.

According to an embodiment, at least one of the plurality of light emitting units may be disposed to correspond to the light receiving unit in a one-to-one or one-to-many manner.

According to an embodiment, the processor may identify a current state of an object to be measured on which the electronic device is worn, may control driving of at least one of the plurality of light emitting units depending on the current state of the object to be measured, and may obtain a light signal through the at least one light receiving unit.

According to an embodiment, the processor may obtain light signals emitted from the first light emitting unit, the second light emitting unit, and the third light emitting unit through the first light receiving unit, the second light receiving unit, and the third light receiving unit when the object to be measured, on which the electronic device is worn, makes a movement exceeding a predetermined distance and may obtain a light signal emitted from the second light emitting unit through the first light receiving unit and the second light receiving unit when the object to be measured makes a movement less than the predetermined distance.

According to an embodiment, the processor may perform control to turn on the first light emitting unit, the second light emitting unit, the third light emitting unit, the first light receiving unit, the second light receiving unit, and the third light receiving unit when the object to be measured, on which the electronic device is worn, makes a movement exceeding a predetermined distance and may perform control to turn off the first light emitting unit, the third light emitting unit, and the third light receiving unit and turn on at least one of the second light emitting unit, the first light receiving unit, or the second light receiving unit when the object to be measured makes a movement less then the predetermined distance.

According to an embodiment, the processor may perform control such that at least one of the first light emitting unit, the second light emitting unit, or the third light emitting unit emits light having a specified first intensity when the object to be measured, on which the electronic device is worn, makes a movement exceeding a predetermined distance and may perform control such that the second light emitting unit emits light having a second intensity lower than the first intensity when the object to be measured, on which the electronic device is worn, makes a movement less than the predetermined distance.

According to an embodiment of the disclosure, an electronic device may include a plurality of light emitting units spaced apart from each other to form a ring shape, at least one light receiving unit disposed between the plurality of light emitting units, and a processor that analyzes light generated from the light emitting units and incident to the light receiving unit. Each of a first one or more light emitting units and a second one or more light emitting units included in the plurality of light emitting units may include at least one first light body that emits light in a specified wavelength band. The first body of the first one or more light emitting units and the first light body of the second one or more light emitting units may have different beam angles. A separation distance between the first light body of the first one or more light emitting units and the light receiving unit may be different from a separation distance between the first light body of the second one or more light emitting units and the light receiving unit.

According to an embodiment, the plurality of light emitting units may include the first one or more light emitting units, the second one or more light emitting units having a smaller beam angle than the first one or more light emitting units, and a third one or more light emitting units having a beam angle smaller than a beam angle of the first one or more light emitting units and greater than a beam angle of the second one or more light emitting units. The at least one light receiving unit may include a first light receiving unit, a second light receiving unit, and a third light receiving unit. The first one or more light emitting units may be disposed between the first light receiving unit and the third light receiving unit and may be spaced apart from the first light receiving unit and the third light receiving unit by a first distance. The second one or more light emitting units may be disposed between the first light receiving unit and the second light receiving unit and may be spaced apart from the first light receiving unit and the second light receiving unit by a second distance different from the first distance. The third one or more light emitting units may be disposed between the second light receiving unit and the third light receiving unit and may be spaced apart from the second light receiving unit and the third light receiving unit by a third distance smaller than the first distance and greater than the second distance.

According to an embodiment, the processor may identify a current state of an object to be measured on which the electronic device is worn, may obtain light signals emitted from the first one or more light emitting units, the second one or more light emitting units, and the third one or more light emitting units through the first light receiving unit, the second light receiving unit, and the third light receiving unit when the object to be measured, on which the electronic device is worn, makes a big movement, and may obtain a light signal emitted from one of the first light emitting units, the second one or more light emitting units, and the third one or more light emitting units through at least two of the first light receiving unit, the second light receiving unit, and the third light receiving unit when the object to be measured makes a little movement.

According to the embodiments of the disclosure, the distances between the light emitting units and the light receiving units may be set to be suitable for the user's body structure, and thus the quality of a biometric signal may be improved.

According to the embodiments of the disclosure, the driving of the light emitting units may be controlled depending on the user's current state, and thus the quality of a biometric signal may be improved.

In addition, according to the embodiments of the disclosure, even though current consumption is lowered, signal quality performance may be maintained by controlling the driving of the light emitting units depending on the user's current state.

The electronic device according to certain embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that certain embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1^{st}" and "2^{nd}," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

According to certain embodiments, each component (e.g., a module or a program) of the above-described components may include a single entity or multiple entities and some of multiple entities may be separately disposed on the other components. According to certain embodiments, one or more of the above-described components may be omitted, or one or more other components may be added. Alternatively or additionally, a plurality of components (e.g., modules or programs) may be integrated into a single component. In such a case, according to certain embodiments, the integrated component may still perform one or more functions of each of the plurality of components in the same or similar manner as they are performed by a corresponding one of the plurality of components before the integration. According to certain embodiments, operations performed by the module, the program, or another component may be carried out sequentially, in parallel, repeatedly, or heuristically, or one or more of the operations may be executed in a different order or omitted, or one or more other operations may be added.

## Claims

1. An electronic device comprising:
a plurality of light emitting units (911, 912, 913) spaced apart from each other on a printed circuit board (901);
a plurality of light receiving units (921, 922, 923) disposed between the plurality of light emitting units to form a ring shape with the plurality of light-emitting units (911, 912, 913); and
a processor (420) configured to analyze light generated from the plurality of light emitting units (911, 912, 913) and incident to the plurality of light receiving units (921, 922, 923),
wherein a separation distance between each of the plurality of light emitting units (911, 912, 913) and the plurality of light receiving units (921, 922, 923) is such that the plurality of light receiving units (921, 922, 923) are configured to receive reflected light from light emitted at a beam angle from each of the plurality of light emitting units (911, 912, 913),
wherein the plurality of light-emitting units (911, 912, 913) include:
a first light-emitting unit (911) having a first beam angle;
a second light-emitting unit (912) having a second beam angle different from the first beam angle; and
a third light-emitting unit (913) having a third beam angle, and
wherein the plurality of light receiving units (921, 922, 923) includes:
a first light-receiving unit (921) disposed at one side of the first light-emitting unit (911) and spaced apart from the first light-emitting unit (921) by a first distance Da;
a second light-receiving unit (922) disposed at one side of the second light-emitting unit (912) and spaced apart from the second light-emitting unit (912) by a second distance Db different from the first distance Da; and
a third light-receiving unit (923) disposed at another side of the first light-emitting unit (911) and spaced apart from the first light-emitting unit (911) by the first distance Da, and
wherein the first distance and the second distance are set based on the respective beam angles of the first light emitting unit and the second light emitting unit.

2. The electronic device of claim 1,
wherein the second light emitting unit is disposed between the first light receiving unit and the second light receiving unit and spaced apart from the first light receiving unit and the second light receiving unit by the second distance,
wherein the third light emitting unit is disposed between the second light receiving unit and the third light receiving unit and spaced apart from the second light receiving unit and the third light receiving unit by a third distance different from the first distance and the second distance, and
wherein the first distance, the second distance, and the third distance are set based on the respective beam angles of the first light emitting unit, the second light emitting unit, and the third light emitting unit.

3. The electronic device of claim 1,
wherein the plurality of light emitting units include a fourth light emitting unit,
wherein the plurality of light receiving units includes a fourth light receiving unit,
wherein the second light emitting unit is disposed between the first light receiving unit and the second light receiving unit,
wherein the third light emitting unit is disposed between the second light receiving unit and the fourth light receiving unit,
wherein the fourth light emitting unit is disposed between the fourth light receiving unit and the third light receiving unit.

4. The electronic device of claim 3, wherein each of the first light emitting unit and the second light emitting unit includes at least one first light body configured to emit light in a specified wavelength band.

5. The electronic device of claim 2, wherein the first light emitting unit has a larger beam angle than the third light emitting unit, and the third light emitting unit has a larger beam angle than the second light emitting unit, and
wherein the first distance is longer than the third distance, and the third distance is shorter than the second distance.

6. The electronic device of claim 2, further comprising:
a first partition wall (730) disposed between the first light emitting unit and the first light receiving unit, a second partition wall (730) disposed between the first light receiving unit and the second light emitting unit, a third partition wall (730) disposed between the second light emitting unit and the second light receiving unit, a fourth partition wall (730) disposed between the second light receiving unit and the third light emitting unit, a fifth partition wall (730) disposed between the third light emitting unit and the third light receiving unit, and a sixth partition wall (730) disposed between the third light receiving unit and the first light emitting unit.

7. The electronic device of claim 1, wherein the second light emitting unit includes:
a light emitting chip configured to generate light in the same wavelength band as the first light emitting unit; and
an optical member disposed over the light emitting chip, and
wherein the optical member is formed of a lens structure or an optical film configured to restrict an emission range of the light generated from the light emitting chip.

8. The electronic device of claim 1, wherein the second light emitting unit includes a light emitting chip configured to generate light in the same wavelength band as the first light emitting unit, the light emitting chip having a smaller light emission range than the first light emitting unit.

9. The electronic device of claim 1, wherein the plurality of light emitting units are disposed to correspond to the plurality of light receiving units in a one-to-one or one-to-many manner.

10. The electronic device of claim 2, wherein the processor:
identifies a current state of an object to be measured on which the electronic device is worn;
controls driving of at least one of the plurality of light emitting units depending on the current state of the object to be measured; and
obtains a light signal through the at least one light receiving unit.

11. The electronic device of claim 10, wherein the processor is configured to:
obtain light signals emitted from the first light emitting unit, the second light emitting unit, and the third light emitting unit through the first light receiving unit, the second light receiving unit, and the third light receiving unit when the object to be measured, on which the electronic device is worn, makes a movement exceeding a predetermined distance; and
obtain a light signal emitted from the second light emitting unit through the first light receiving unit and the second light receiving unit when the object to be measured makes a movement less than the predetermined distance.

12. The electronic device of claim 11, wherein the processor:
performs control to turn on the first light emitting unit, the second light emitting unit, the third light emitting unit, the first light receiving unit, the second light receiving unit, and the third light receiving unit when the object to be measured, on which the electronic device is worn, makes a movement exceeding the predetermined distance; and
performs control to turn off the first light emitting unit, the third light emitting unit, and the third light receiving unit and turn on at least one of the second light emitting unit, the first light receiving unit, or the second light receiving unit when the object to be measured makes a movement less than the predetermined distance.

13. The electronic device of claim 12, wherein the processor:
performs control such that at least one of the first light emitting unit, the second light emitting unit, or the third light emitting unit emits light having a specified first intensity when the object to be measured, on which the electronic device is worn, makes a movement exceeding the predetermined distance; and
performs control such that the second light emitting unit emits light having a second intensity lower than the first intensity when the object to be measured, on which the electronic device is worn, makes a movement less than the predetermined distance.

## Patentansprüche

1. Elektronische Vorrichtung, umfassend:
eine Vielzahl von Lichtemissionseinheiten (911, 912, 913), die voneinander beabstandet auf einer Leiterplatte (901) angeordnet sind;
eine Vielzahl von Lichtempfangseinheiten (921, 922, 923), die zwischen der Vielzahl von Lichtemissionseinheiten angeordnet sind, um mit der Vielzahl von Lichtemissionseinheiten (911, 912, 913) eine Ringform zu bilden; und
einen Prozessor (420), der dazu konfiguriert ist, von der Vielzahl von Lichtemissionseinheiten (911, 912, 913) erzeugtes und auf die Vielzahl von Lichtempfangseinheiten (921, 922, 923) einfallendes Licht zu analysieren,
wobei ein Trennungsabstand zwischen jeder der Vielzahl von Lichtemissionseinheiten (911, 912, 913) und der Vielzahl von Lichtempfangseinheiten (921, 922, 923) so bemessen ist, dass die Vielzahl von Lichtempfangseinheiten (921, 922, 923) dazu konfiguriert sind, reflektiertes Licht von Licht zu empfangen, das in einem Strahlwinkel von jeder der Vielzahl von Lichtemissionseinheiten (911, 912, 913) emittiert wird,
wobei die Vielzahl von Lichtemissionseinheiten (911, 912, 913) Folgendes umfassen:
eine erste Lichtemissionseinheit (911) mit einem ersten Strahlwinkel;
eine zweite Lichtemissionseinheit (912) mit einem zweiten Strahlwinkel, der sich vom ersten Strahlwinkel unterscheidet; und
eine dritte Lichtemissionseinheit (913) mit einem dritten Strahlwinkel, und
wobei die Vielzahl von Lichtempfangseinheiten (921, 922, 923) Folgendes umfassen:
eine erste Lichtempfangseinheit (921), die an einer Seite der ersten Lichtemissionseinheit (911) angeordnet und von der ersten Lichtemissionseinheit (921) um einen ersten Abstand Da beabstandet ist;
eine zweite Lichtempfangseinheit (922), die an einer Seite der zweiten Lichtemissionseinheit (912) angeordnet und von der zweiten Lichtemissionseinheit (912) um einen zweiten Abstand Db beabstandet ist, der sich vom ersten Abstand Da unterscheidet; und
eine dritte Lichtempfangseinheit (923), die an einer weiteren Seite der ersten Lichtemissionseinheit (911) angeordnet und von der ersten Lichtemissionseinheit (911) um den ersten Abstand Da beabstandet ist, und
wobei der erste Abstand und der zweite Abstand auf der Grundlage der jeweiligen Strahlwinkel der ersten Lichtemissionseinheit und der zweiten Lichtemissionseinheit eingestellt werden.

2. Elektronische Vorrichtung nach Anspruch 1,
wobei die zweite Lichtemissionseinheit zwischen der ersten Lichtempfangseinheit und der zweiten Lichtempfangseinheit angeordnet und von der ersten Lichtempfangseinheit und der zweiten Lichtempfangseinheit um den zweiten Abstand beabstandet ist,
wobei die dritte Lichtemissionseinheit zwischen der zweiten Lichtempfangseinheit und der dritten Lichtempfangseinheit angeordnet und von der zweiten Lichtempfangseinheit und der dritten Lichtempfangseinheit um einen dritten Abstand beabstandet ist, der sich von dem ersten Abstand und dem zweiten Abstand unterscheidet, und
wobei der erste Abstand, der zweite Abstand und der dritte Abstand auf der Grundlage der jeweiligen Strahlwinkel der ersten Lichtemissionseinheit, der zweiten Lichtemissionseinheit und der dritten Lichtemissionseinheit eingestellt werden.

3. Elektronische Vorrichtung nach Anspruch 1,
wobei die Vielzahl von Lichtemissionseinheiten eine vierte Lichtemissionseinheit umfassen,
wobei die Vielzahl von Lichtempfangseinheiten eine vierte Lichtempfangseinheit umfassen,
wobei die zweite Lichtemissionseinheit zwischen der ersten Lichtempfangseinheit und der zweiten Lichtempfangseinheit angeordnet ist,
wobei die dritte Lichtemissionseinheit zwischen der zweiten Lichtempfangseinheit und der vierten Lichtempfangseinheit angeordnet ist,
wobei die vierte Lichtemissionseinheit zwischen der vierten Lichtempfangseinheit und der dritten Lichtempfangseinheit angeordnet ist.

4. Elektronische Vorrichtung nach Anspruch 3, wobei jede von der ersten Lichtemissionseinheit und der zweiten Lichtemissionseinheit mindestens einen ersten Lichtkörper umfasst, der dazu konfiguriert ist, Licht in einem vorgegebenen Wellenlängenband zu emittieren.

5. Elektronische Vorrichtung nach Anspruch 2, wobei die erste Lichtemissionseinheit einen größeren Strahlwinkel als die dritte Lichtemissionseinheit und die dritte Lichtemissionseinheit einen größeren Strahlwinkel als die zweite Lichtemissionseinheit aufweist, und
wobei der erste Abstand größer als der dritte Abstand und der dritte Abstand kleiner als der zweite Abstand ist.

6. Elektronische Vorrichtung nach Anspruch 2, umfassend ferner:
eine erste Trennwand (730), die zwischen der ersten Lichtemissionseinheit und der ersten Lichtempfangseinheit angeordnet ist, eine zweite Trennwand (730), die zwischen der ersten Lichtempfangseinheit und der zweiten Lichtemissionseinheit angeordnet ist, eine dritte Trennwand (730), die zwischen der zweiten Lichtemissionseinheit und der zweiten Lichtempfangseinheit angeordnet ist, eine vierte Trennwand (730), die zwischen der zweiten Lichtempfangseinheit und der dritten Lichtemissionseinheit angeordnet ist, eine fünfte Trennwand (730), die zwischen der dritten Lichtemissionseinheit und der dritten Lichtempfangseinheit angeordnet ist, und eine sechste Trennwand (730), die zwischen der dritten Lichtempfangseinheit und der ersten Lichtemissionseinheit angeordnet ist.

7. Elektronische Vorrichtung nach Anspruch 1, wobei die zweite Lichtemissionseinheit Folgendes umfasst:
einen Lichtemissionschip, der dazu konfiguriert ist, Licht im gleichen Wellenlängenband wie die erste Lichtemissionseinheit zu erzeugen; und
ein optisches Element, das über dem Lichtemissionschip angeordnet ist, und
wobei das optische Element aus einer Linsenstruktur oder einem optischen Film gebildet ist, die bzw. der dazu konfiguriert ist, den Emissionsbereich des vom Lichtemissionschip erzeugten Lichts einzuschränken.

8. Elektronische Vorrichtung nach Anspruch 1, wobei die zweite Lichtemissionseinheit einen Lichtemissionschip umfasst, der dazu konfiguriert ist, Licht im gleichen Wellenlängenband wie die erste Lichtemissionseinheit zu erzeugen, wobei der Lichtemissionschip einen kleineren Lichtemissionsbereich als die erste Lichtemissionseinheit aufweist.

9. Elektronische Vorrichtung nach Anspruch 1, wobei die Vielzahl von Lichtemissionseinheiten so angeordnet sind, dass sie den Vielzahl von Lichtempfangseinheiten in einer Eins-zu-Eins- oder Eins-zu-Viele-Weise entsprechen.

10. Elektronische Vorrichtung nach Anspruch 2, wobei der Prozessor:
einen aktuellen Zustand eines zu messenden Objekts identifiziert, an dem die elektronische Vorrichtung getragen wird;
den Antrieb mindestens einer der Vielzahl von Lichtemissionseinheiten in Abhängigkeit vom aktuellen Zustand des zu messenden Objekts steuert; und
ein Lichtsignal über die mindestens eine Lichtempfangseinheit erhält.

11. Elektronische Vorrichtung nach Anspruch 10, wobei der Prozessor dazu konfiguriert ist:
Lichtsignale, die von der ersten Lichtemissionseinheit, der zweiten Lichtemissionseinheit und der dritten Lichtemissionseinheit emittiert werden, über die erste Lichtempfangseinheit, die zweite Lichtempfangseinheit und die dritte Lichtempfangseinheit zu erhalten, wenn das zu messende Objekt, an dem die elektronische Vorrichtung getragen wird, eine Bewegung durchführt, die eine vorbestimmte Entfernung überschreitet; und
ein von der zweiten Lichtemissionseinheit emittiertes Lichtsignal über die erste Lichtempfangseinheit und die zweite Lichtempfangseinheit zu erhalten, wenn das zu messende Objekt eine Bewegung von weniger als der vorbestimmten Entfernung durchführt.

12. Elektronische Vorrichtung nach Anspruch 11, wobei der Prozessor:
eine Steuerung durchführt, um die erste Lichtemissionseinheit, die zweite Lichtemissionseinheit, die dritte Lichtemissionseinheit, die erste Lichtempfangseinheit, die zweite Lichtempfangseinheit und die dritte Lichtempfangseinheit einzuschalten, wenn das zu messende Objekt, an dem die elektronische Vorrichtung getragen wird, eine Bewegung durchführt, die die vorbestimmte Entfernung überschreitet; und
eine Steuerung durchführt, um die erste Lichtemissionseinheit, die dritte Lichtemissionseinheit und die dritte Lichtempfangseinheit auszuschalten und mindestens eine von der zweiten Lichtemissionseinheit, der ersten Lichtempfangseinheit und der zweiten Lichtempfangseinheit einzuschalten, wenn das zu messende Objekt eine Bewegung von weniger als der vorbestimmten Entfernung durchführt.

13. Elektronische Vorrichtung nach Anspruch 12, wobei der Prozessor:
eine Steuerung durchführt, so dass mindestens eine von der ersten Lichtemissionseinheit, der zweiten Lichtemissionseinheit und der dritten Lichtemissionseinheit Licht mit einer vorgegebenen ersten Intensität emittiert, wenn das zu messende Objekt, an dem die elektronische Vorrichtung getragen wird, eine Bewegung durchführt, die die vorbestimmte Entfernung überschreitet; und
eine Steuerung durchführt, so dass die zweite Lichtemissionseinheit Licht mit einer zweiten Intensität emittiert, die geringer ist als die erste Intensität, wenn das zu messende Objekt, an dem die elektronische Vorrichtung getragen wird, eine Bewegung von weniger als der vorbestimmten Entfernung durchführt.

## Revendications

1. Dispositif électronique comprenant :
une pluralité d'unités électroluminescentes (911, 912, 913) espacées les unes des autres sur une carte de circuit imprimé (901) ;
une pluralité d'unités de réception de lumière (921, 922, 923) disposées entre la pluralité d'unités électroluminescentes pour former une forme annulaire avec la pluralité d'unités électroluminescentes (911, 912, 913) ; et
un processeur (420) configuré pour analyser la lumière générée par la pluralité d'unités électroluminescentes (911, 912, 913) et incidente sur la pluralité d'unités de réception de lumière (921, 922, 923),
dans lequel une distance de séparation entre chacune de la pluralité d'unités électroluminescentes (911, 912, 913) et la pluralité d'unités de réception de lumière (921, 922, 923) est telle que la pluralité d'unités de réception de lumière (921, 922, 923) sont configurées pour recevoir une lumière réfléchie provenant de la lumière émise selon un angle de faisceau par chacune de la pluralité d'unités électroluminescentes (911, 912, 913),
dans lequel la pluralité d'unités électroluminescentes (911, 912, 913) comprennent :
une première unité électroluminescente (911) ayant un premier angle de faisceau ;
une deuxième unité électroluminescente (912) ayant un deuxième angle de faisceau différent du premier angle de faisceau ; et
une troisième unité électroluminescente (913) ayant un troisième angle de faisceau, et
dans lequel la pluralité d'unités de réception de lumière (921, 922, 923) comprend :
une première unité de réception de lumière (921) disposée d'un côté de la première unité électroluminescente (911) et espacée de la première unité électroluminescente (921) d'une première distance Da ;
une deuxième unité de réception de lumière (922) disposée d'un côté de la deuxième unité électroluminescente (912) et espacée de la deuxième unité électroluminescente (912) d'une deuxième distance Db différente de la première distance Da ; et
une troisième unité de réception de lumière (923) disposée d'un autre côté de la première unité électroluminescente (911) et espacée de la première unité électroluminescente (911) de la première distance Da, et
dans lequel la première distance et la deuxième distance sont définies sur la base des angles de faisceau respectifs de la première unité électroluminescente et de la deuxième unité électroluminescente.

2. Dispositif électronique selon la revendication 1,
dans lequel la deuxième unité électroluminescente est disposée entre la première unité de réception de lumière et la deuxième unité de réception de lumière et espacée de la première unité de réception de lumière et de la deuxième unité de réception de lumière de la deuxième distance,
dans lequel la troisième unité électroluminescente est disposée entre la deuxième unité de réception de lumière et la troisième unité de réception de lumière et espacée de la deuxième unité de réception de lumière et de la troisième unité de réception de lumière d'une troisième distance différente de la première distance et de la deuxième distance, et
dans lequel la première distance, la deuxième distance et la troisième distance sont définies sur la base des angles de faisceau respectifs de la première unité électroluminescente, de la deuxième unité électroluminescente et de la troisième unité électroluminescente.

3. Dispositif électronique selon la revendication 1,
dans lequel la pluralité d'unités électroluminescentes comprennent une quatrième unité électroluminescente,
dans lequel la pluralité d'unités de réception de lumière comprend une quatrième unité de réception de lumière,
dans lequel la deuxième unité électroluminescente est disposée entre la première unité de réception de lumière et la deuxième unité de réception de lumière,
dans lequel la troisième unité électroluminescente est disposée entre la deuxième unité de réception de lumière et la quatrième unité de réception de lumière,
dans lequel la quatrième unité électroluminescente est disposée entre la quatrième unité de réception de lumière et la troisième unité de réception de lumière.

4. Dispositif électronique selon la revendication 3, dans lequel chacune de la première unité électroluminescente et de la deuxième unité électroluminescente comprend au moins un premier corps lumineux configuré pour émettre de la lumière dans une bande de longueurs d'onde spécifiée.

5. Dispositif électronique selon la revendication 2, dans lequel la première unité électroluminescente a un angle de faisceau plus grand que la troisième unité électroluminescente, et la troisième unité électroluminescente a un angle de faisceau plus grand que la deuxième unité électroluminescente, et
dans lequel la première distance est plus longue que la troisième distance, et la troisième distance est plus courte que la deuxième distance.

6. Dispositif électronique selon la revendication 2, comprenant en outre :
une première paroi de séparation (730) disposée entre la première unité électroluminescente et la première unité de réception de lumière, une deuxième paroi de séparation (730) disposée entre la première unité de réception de lumière et la deuxième unité électroluminescente, une troisième paroi de séparation (730) disposée entre la deuxième unité électroluminescente et la deuxième unité de réception de lumière, une quatrième paroi de séparation (730) disposée entre la deuxième unité de réception de lumière et la troisième unité électroluminescente, une cinquième paroi de séparation (730) disposée entre la troisième unité électroluminescente et la troisième unité de réception de lumière, et une sixième paroi de séparation (730) disposée entre la troisième unité de réception de lumière et la première unité électroluminescente.

7. Dispositif électronique selon la revendication 1, dans lequel la deuxième unité électroluminescente comprend :
une puce électroluminescente configurée pour générer de la lumière dans la même bande de longueurs d'onde que la première unité électroluminescente ; et
un élément optique disposé sur la puce électroluminescente, et
dans lequel l'élément optique est formé d'une structure de lentille ou d'un film optique configuré pour restreindre une plage d'émission de la lumière générée par la puce électroluminescente.

8. Dispositif électronique selon la revendication 1, dans lequel la deuxième unité électroluminescente comprend une puce électroluminescente configurée pour générer de la lumière dans la même bande de longueurs d'onde que la première unité électroluminescente, la puce électroluminescente ayant une plage d'émission de lumière plus petite que la première unité électroluminescente.

9. Dispositif électronique selon la revendication 1, dans lequel la pluralité d'unités électroluminescentes sont disposées pour correspondre à la pluralité d'unités de réception de lumière de manière un à un ou un à plusieurs.

10. Dispositif électronique selon la revendication 2, dans lequel le processeur :
identifie un état actuel d'un objet à mesurer sur lequel le dispositif électronique est porté ;
commande l'entraînement d'au moins l'une de la pluralité d'unités électroluminescentes en fonction de l'état actuel de l'objet à mesurer ; et
obtient un signal lumineux par l'intermédiaire d'au moins une unité de réception de lumière.

11. Dispositif électronique selon la revendication 10, dans lequel le processeur est configuré pour :
obtenir des signaux lumineux émis par la première unité électroluminescente, la deuxième unité électroluminescente et la troisième unité électroluminescente par l'intermédiaire de la première unité de réception de lumière, de la deuxième unité de réception de lumière et de la troisième unité de réception de lumière lorsque l'objet à mesurer, sur lequel le dispositif électronique est porté, effectue un mouvement dépassant une distance prédéterminée ; et
obtenir un signal lumineux émis par la deuxième unité électroluminescente par l'intermédiaire de la première unité de réception de lumière et de la deuxième unité de réception de lumière lorsque l'objet à mesurer effectue un mouvement inférieur à la distance prédéterminée.

12. Dispositif électronique selon la revendication 11, dans lequel le processeur :
effectue une commande pour allumer la première unité électroluminescente, la deuxième unité électroluminescente, la troisième unité électroluminescente, la première unité de réception de lumière, la deuxième unité de réception de lumière et la troisième unité de réception de lumière lorsque l'objet à mesurer, sur lequel le dispositif électronique est porté, effectue un mouvement dépassant la distance prédéterminée ; et
effectue une commande pour éteindre la première unité électroluminescente, la troisième unité électroluminescente et la troisième unité de réception de lumière et allumer au moins l'une parmi la deuxième unité électroluminescente, la première unité de réception de lumière ou la deuxième unité de réception de lumière lorsque l'objet à mesurer effectue un mouvement inférieur à la distance prédéterminée.

13. Dispositif électronique selon la revendication 12, dans lequel le processeur :
effectue une commande de sorte qu'au moins l'une parmi la première unité électroluminescente, la deuxième unité électroluminescente ou la troisième unité électroluminescente émette de la lumière ayant une première intensité spécifiée lorsque l'objet à mesurer, sur lequel le dispositif électronique est porté, effectue un mouvement dépassant la distance prédéterminée ; et
effectue une commande de sorte que la deuxième unité électroluminescente émette de la lumière ayant une deuxième intensité inférieure à la première intensité lorsque l'objet à mesurer, sur lequel le dispositif électronique est porté, effectue un mouvement inférieur à la distance prédéterminée.
